(19) Europäisches Patentamt
European Patent Office
Office européen des brevets

(11) Veröffentlichungsnummer: **0 132 769**
**B1**

(12) # EUROPÄISCHE PATENTSCHRIFT

(45) Veröffentlichungstag der Patentschrift: **07.01.88**

(51) Int. Cl.⁴: **A 61 K 37/26**

(21) Anmeldenummer: **84108441.1**

(22) Anmeldetag: **18.07.84**

(54) **Pharmazeutisches Mittel zur Behandlung des Diabetes mellitus.**

(30) Priorität: **22.07.83 DE 3326473**

(43) Veröffentlichungstag der Anmeldung: **13.02.85 Patentblatt 85/7**

(45) Bekanntmachung des Hinweises auf die Patenterteilung: **07.01.88 Patentblatt 88/1**

(84) Benannte Vertragsstaaten: **AT BE CH DE FR GB IT LI LU NL SE**

(56) Entgegenhaltungen:
**EP - A - 0 092 280**
**FR - A - 2 511 867**
**GB - A - 2 104 380**
**GB - A - 2 104 382**

(73) Patentinhaber: **HOECHST AKTIENGESELLSCHAFT, Postfach 80 03 20, D-6230 Frankfurt am Main 80 (DE)**

(72) Erfinder: **Grau, Ulrich, Dr., Zeil 17, D-6238 Hofheim am Taunus (DE)**

**EP 0 132 769 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Diabetes mellitus ist eine Stoffwechselstörung, die als wesentliches Symptom einen erhöhten Blutzuckerspiegel zeigt. Sie hat ihre Ursache darin, dass das pankreatische Hormon Insulin nicht in genügender Menge freigesetzt wird. Die Substitution des natürlichen Hormons geschieht heute in der Regel durch tierisches Insulin, das aus Schlachttierdrüsen isoliert wird oder humanes Insulin, das semisynthetisch aus Schweineinsulin oder durch gentechnologische Methoden zugänglich ist.

Bei der Anwendung gentechnologischer Methoden wurden bisher zwei grundsätzlich verschiedene Wege eingeschlagen: die getrennte Synthese von A- und B-Ketten und deren nachträgliche chemische Rekombination, sowie die Synthese von Präproinsulin, des natürlichen Vorläufers des Insulins. Im Proinsulin-Molekül sind A- und B-Kette durch ein Verbindungsstück, das C-Peptid, verknüpft. Dessen wichtigste Funktion ist nach gegenwärtiger Vorstellung die räumliche Fixierung der beiden Ketten relativ zueinander, so dass eine korrekte Faltung stattfinden kann. Nachdem die Faltung erfolgt ist, werden die drei Disulfidbrücken geknüpft und damit die native dreidimensionale Struktur des Insulins stabilisiert. Die Abspaltung des C-Peptids erfolgt durch Enzyme mit tryptischer und Carboxypeptidase-B-Aktivität. Die Spaltstellen sind durch eine Sequenz Lys-Arg (vor dem N-Terminus der A-Kette) bzw. Arg-Arg (am C-Terminus der B-Kette) vorgegeben. Nur das freie Insulin besitzt volle biologische Aktivität, weil bei Gegenwart des C-Peptids wahrscheinlich ein Teil der biologischen Erkennungsregion an der Oberfläche des Moleküls maskiert ist.

Die spezielle chemische Natur des Insulins bringt es mit sich, dass die Therapie in der Regel parenteral erfolgt; das Hormon würde z.B. bei Magen/Darm-Passage vollständig abgebaut, noch bevor es zur Wirkung kommen kann. Abbaureaktionen, im wesentlichen durch verschiedene, relativ unspezifische proteolytische Enzyme, finden aber auch am Injektionsort und in der Zirkulation statt. Die dadurch bedingte kurze Halbwertszeit in vivo von nur etwa 7 Minuten ist physiologisch im Sinne einer Homöostase im Grunde sinnvoll; die Therapie wird dadurch aber erheblich erschwert, weil der Diabetiker typischerweise viermal täglich, in der Regel kurz vor den Mahlzeiten, spritzen muss.

Es hat demgeäss schon frühzeitig Versuche gegeben, dem Insulin ein protrahierte Wirkung zu verleihen. Am erfolgreichsten waren dabei bisher solche Methoden, bei denen durch Zusatz eines Depothilfsstoffes das Insulin in eine schwerlösliche Zustandsform überführt wird. Dazu zählen vor allem zweiwertige Zinkionen, in deren Gegenwart das Insulin in neutralem Medium in kristalliner oder amorpher Form vorliegen kann. Der Zusatz von basischen Proteinen, z.B. Protaminsulfat oder Humanglobin, hat, nachdem Insulin ein saures Molekül vom isoelektrischen Punkt $p_I = 5{,}4$ ist, den gleichen Effekt: basisches Protein und Insulin liegen im Neutralbereich als kristalliner oder amorpher salzartiger, schwerlöslicher Komplex vor.

Die langsame Freisetzung des Insulins aus diesen Retardzubereitungen erfolgt, wie man sich vorstellt, durch Verdünnung, d.h. Diffusion einzelner, den Kristall oder den amorphen Niederschlag aufbauenden Komponenten oder, im Fall von Insulin-Komplexen mit basischen Proteinen, durch protoeolytischen Abbau des Depotträgers.

Neuerdings wird noch humanes Proinsulin, entweder allein oder in Kombination mit den üblichen Depotzusätzen, als Verzögerungsprinzip diskutiert (s. DE-A 3 232 036). Die Vorstellung ist, dass die proteolytische Abspaltung des C-Peptids in vivo verzögert erfolgt, und damit aus dem an sich biologisch nur wenig aktiven Proinsulin (ca. 1/8 der Aktivität des Insulins, bezogen auf Proteinmenge) das voll aktive Hormon freigesetzt wird. Nur solche Proinsuline sind für die Anwendung am Menschen akzeptabel, die in ihrer Sequenz jener des Menschen identisch (davon gibt es offenbar mehrere) oder sehr ähnlich sind. Schweine- oder Rinderproinsulin sind, wie allgemein bekannt, immunogen. Die exakte Wirkungsweise des Proinsulins bleibt derzeit jedoch noch offen. Es gilt keineswegs als erwiesen, dass spezifisch Insulin freigesetzt wird. Im Gegenteil, der Abbau in vivo wird in vielfältiger Weise unter Produktion zumeist inaktiver Bruchstücke erfolgen. Der therapeutische Nutzen des Proinsulins könnte also vielmehr, wenn überhaupt, auf der Rezeptorebene zu suchen sein.

Nun ist die Diabetestherapie gekennzeichnet von individuellen Einflussfaktoren, wie Unterschiede in der Verwertbarkeit der Mahlzeiten, Unterschiede in der Charakteristik des Unterhautgewebes, daneben aber auch die spezifischen Essgewohnheiten, körperliche Aktivitäten und viele mehr. Es ist somit unabdingbar für eine gute Blutzuckereinstellung, eine Reihe von Insulinpräparaten mit unterschiedlicher Wirkungscharakteristik zur Verfügung zu haben, die den individuellen Bedürfnissen angepasst sind. Im Zusammenhang mit nichtoptimaler Einstellung werden neben den unmittelbaren subjektiven und objektiven Effekten, wie Hyper- oder Hypoglykämien, insbesondere der Formenkreis der diabetischen Spätschäden, diskutiert. Dazu zählen vor allem Makro- und Mikroangiopathie, Neuropathie, Nephropathie und Retinopathie.

Als Präparationen, die den Bedürfnissen der Patienten optimal angepasst sind, haben sich neben reinen Verzögerungsinsulinen vor allem sogenannte Intermediärinsuline erwiesen. Sie stellen Mischungen aus einer verzögert und einer sofort und kurz wirksamen Komponente dar. Solche Mischungen stellen i.a. komplizierte Mehrphasensysteme dar, die über lange Zeit hinweg nur in relativ eng begrenzten Mischungsverhältnissen stabil bleiben. So ist beispielsweise eine Suspension von 2-Zink-Insulinkristallen vom Schwein nicht frei mischbar mit gelöstem Schweineinsulin. Sofort oder im Lauf der Zeit fällt

das zugemischte, gelöste Insulin wegen des relativ hohen Zinkgehalts, der zur Stabilisierung der Kristalle nötig ist, aus. Solche Mischungen sind innerhalb enger Grenzen stabil, wenn als gelöstes Insulin Rinderinsulin (hiermit wird jedoch die Speziesreinheit, eine medizinisch erwünschte Eigenschaft, eingebüsst) oder eine Mischung aus gelöstem Schweineinsulin und des Phenylalanin(B1)-Schweineinsulin verwendet wird (DE-A 2 418 218 und DE-A 2 459 515). Vorteilhafter hinsichtlich der Mischbarkeit mit gelöstem Insulin sind Protamin-Insulin-Zubereitungen, wenn als Verzögerungskomponente Kristalle aus Protamin und Insulin im isophanen Verhältnis eingesetzt werden. Mit diesen Präparaten sind NPH-typische Wirkprofile herstellbar; die Gegenwart des Protaminsulfats als körperfremdes, jedoch relativ unbedenkliches Protein als Zusatz erscheint vertretbar.

Aufgabe der Erfindung ist die Bereitstellung eines stabilen pharmazeutischen Mittels, das eine den individuellen Bedürfnissen des Diabetikers angepasste Wirkungscharakteristik aufweist.

Diese Aufgabe wurde nun erfindungsgemäss gelöst durch eine Wirkstoffkombination aus einem Insulin-Derivat, dessen B-Kette C-terminal eine organische Gruppe basischen Charakters trägt, und einem nativen Insulin bzw. seinem Des-Phe$^{B31}$-Analogen.

Insulin-Derivate, die am C-terminalen Ende der B-Kette die Reste Arg-OH oder Arg-Arg-OH tragen wurden schon beschrieben. Bekanntlich entstehen diese Derivate bei der enzymatischen Umwandlung von Proinsulin in Insulin in vivo als natürliche Zwischenprodukte und sind auch in kleinen Anteilen in Pankreasextrakten nachweisbar. Die genannten Reste werden normalerweise durch Trypsin und/oder Carboxypeptidase B oder Enzyme mit ähnlicher Spezifität und Freisetzung des nativen Insulins abgespalten.

Weitere dieser C-terminal basisch modifizierten Insulin-Derivate, Verfahren zu ihrer Herstellung und ihre Verwendung sind Gegenstand der EP-A 84 108 442.

Die Erfindung betrifft Arzneimittel aus einem physiologisch unbedenklichen Träger und einer Wirkstoffkombination, die dadurch gekennzeichnet sind, dass sie als Wirkstoffkombination

a) ein Insulin-Derivat der Formel I,

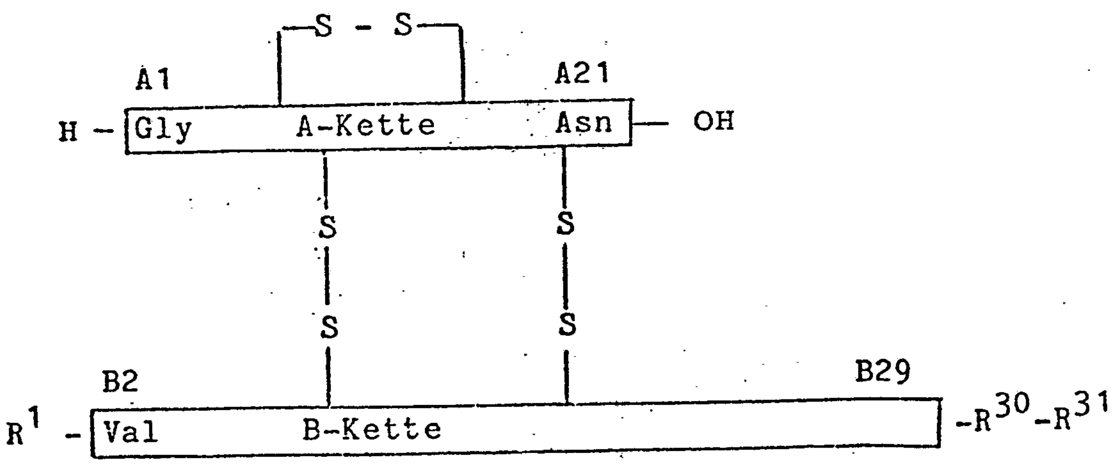


(I)

in welcher

$R^1$ H oder H-Phe bedeutet,

$R^{30}$ für den Rest einer neutralen, genetisch codierbaren L-Aminosäure steht und

$R^{31}$ für eine physiologisch verträgliche organische Gruppe basischen Charakters mit bis zu 50 C-Atomen steht, an deren Aufbau 0 bis 3 $\alpha$-Aminosäuren beteiligt sind und deren gegebenenfalls vorhandene endständige Carboxyfunktion frei, als Esterfunktion, als Amidfunktion, als Lacton oder zu $CH_2OH$ reduziert vorliegen kann,

mit einem isoelektrischen Punkt zwischen 5,8 und 8,5 und

$b_1$) ein Insulin der Formel I, in welcher $R^1$ H oder H-Phe bedeutet, $R^{30}$ für Ala, Thr oder Ser steht und $R^{31}$ OH bedeutet, oder

$b_2$) physiologisch unbedenkliche Salze davon, oder

$b_3$) Proinsulin oder

$b_4$) C-Peptid oder

$b_5$) eine Kombination von $b_3$) und $b_4$) enthält,

wobei Komponente a) in einer Menge von mindestens 1% zugegen ist.

Bevorzugt sind solche, die dadurch gekennzeichnet sind, dass in dem unter a) genannten Insulin-Derivat der Formel I

$R^{31}$ für den Rest der Formel $-X_n-S$ steht,

in welchem

$n = 0, 1, 2$ oder 3 ist,

X für gleiche oder verschiedene Reste natürlich vorkommender neutraler oder basischer L-Aminosäuren (vorzugsweise basischer L-Aminosäure, insbesondere Arg, Lys, His oder Orn) und/oder der diesen entsprechenden D-Aminosäuren steht und S OH oder eine physiologisch verträgliche, die Carboxygruppe blockierende Gruppe bedeutet, die, falls $n = 0$ ist, einen positiv geladenen

oder protonierbaren basischen Rest trägt oder, falls $n > 0$ ist, einen solchen Rest tragen kann und worin der C-Terminus
-X-S auch für den Rest einer zum entsprechenden Alkohol reduzierten Aminosäure oder, im Falle $n = 2$ oder 3, für den Homoserinlacton-Rest stehen kann.

Insulin-Derivate der Formel I, die in Position B1 Phenylalanin tragen, sind besonders bevorzugt. Weiterhin sind solche bevorzugt, die in der Position B30 Ala, Thr oder Ser aufweisen. Ihre A-Kette und die Kette (B 2-29) weist zweckmässigerweise die Sequenzen des Rinder- oder Schweineinsulins, insbesondere aber die des Humaninsulins auf.

Die Aminosäurereste X und Reste der entsprechenden Derivate können unabhängig voneinander in der D- oder L-Konfiguration vorliegen. Bevorzugt ist jedoch die L-Konfiguration aller Reste.

Genetisch kodierbar sind die folgenden L-Aminosäuren: Gly, Ala, Ser, Thr, Val, Leu, Ile, Asp, Asn, Glu, Gln, Cys, Met, Arg, Lys, His, Tyr, Phe, Trp, Pro (neutrale Aminosäuren unterstrichen).

Unter einer neutralen, natürlich vorkommenden Aminosäure versteht man insbesondere Gly, Ala, Ser, Thr, Val, Leu, Ile, Asn, Gln, Cys, Met, Tyr, Phe, Pro oder Hyp. Unter einer basischen, natürlich vorkommenden Aminosäure versteht man insbesondere Arg, Lys, Hyl, Orn, Cit oder His.

Unter Gruppen, die gegebenenfalls eine freie Carboxyfunktion am C-terminalen Ende der B-Kette in den erfindungsgemässen Insulin-Derivaten blockieren, versteht man vor allem Ester- und Amidgruppen, vorzugsweise ($C_1$ bis $C_6$)-Alkoxy, ($C_3$ bis $C_6$)-Cycloalkyloxy, $NH_2$, ($C_1$ bis $C_6$)-Alkylamino oder Di-($C_1$ bis $C_6$)-alkylamino, oder basische Gruppen, wie Amino-($C_2$ bis $C_6$)-alkoxy, ($C_2$ bis $C_4$)-Alkylamino-($C_2$ bis $C_6$)-alkoxy, Di-($C_1$ bis $C_4$)-alkylamino-($C_2$ bis $C_6$)-alkoxy, Tri-($C_1$ bis $C_4$alkyl)-ammonio-($C_2$ bis $C_6$) alkoxy, Amino-($C_2$ bis $C_6$)-alkylamino, ($C_1$ bis $C_4$)-Alkylamino)-($C_2$ bis $C_6$)-alkylamino, [Di-($C_1$ bis $C_4$)-alkylamino]-($C_2$ bis $C_6$)-alkylamino oder [Tri($C_1$ bis $C_4$)alkylammonio]-($C_2$ bis $C_6$)-alkylamino, insbesondere $-O-(CH_2)_p-NR_2$, $-O-(CH_2)_p-\overset{\oplus}{N}R_3$, $-NH-(CH_2)_p-NR_2$ oder $-NH-(CH_2)_p-\overset{\oplus}{N}R_3$, worin $p = 2$ bis 6 ist und R gleich oder verschieden ist und für Wasserstoff oder ($C_1$ bis $C_4$)-Alkyl steht.

Aus der Reihe der erfindungsgemässen Insulin-Derivate der Formel I seien beispielsweise die nachstehenden Verbindungen erwähnt, ohne die Erfindung auf diese zu beschränken:

Humaninsulin-Arg$^{B31}$-OH
Schweineinsulin-Arg$^{B31}$-OH
Rinderinsulin-Arg$^{B31}$-OH
Humaninsulin-Arg$^{B31}$-Arg$^{B32}$-OH
Schweineinsulin-Arg$^{B31}$-Arg$^{B32}$-OH
Rinderinsulin-Arg$^{B31}$-Arg$^{B32}$-OH
Des-Phe$^{B1}$-Schweineinsulin-Arg$^{B31}$-OH
Des-Phe$^{B1}$-Humaninsulin-Arg$^{B31}$-OH
Des-Phe$^{B1}$-Schweineinsulin-Arg$^{B31}$-Arg$^{B32}$-OH
Des-Phe$^{B1}$-Humaninsulin-Arg$^{B31}$-Arg$^{B32}$-OH
Schweineinsulin-Arg$^{B31}$-$OCH_3$
Humaninsulin-Arg$^{B31}$-$OCH_3$
Rinderinsulin-Arg$^{B31}$-$OCH_3$
Schweineinsulin-Arg$^{B31}$-Arg$^{B32}$-$OCH_3$
Humaninsulin-Arg$^{B31}$-Arg$^{B32}$-$OCH_3$
Des-Thr$^{B30}$-Humaninsulin-Val$^{B30}$-Arg$^{B31}$-$OCH_3$
Des-Thr$^{B30}$-Humaninsulin-Val$^{B30}$-Arg$^{B31}$-OH
Des-Thr$^{B30}$-Humaninsulin-Val$^{B30}$-Ala$^{B31}$-Arg$^{B32}$-OH
Humaninsulin-Lys$^{B31}$-OH
Humaninsulin-D-Arg$^{B31}$-OH
Humaninsulin-D-Arg$^{B31}$-Arg$^{B32}$-OH
Humaninsulin-Arg$^{B31}$-D-Arg$^{B32}$-OH
Humaninsulin-Lys-$^{B31}$-Arg$^{B32}$-OH
Humaninsulin-Arg$^{B31}$-Lys$^{B32}$-OH
Humaninsulin-Argininol$^{B31}$
Humaninsulin-Val$^{B31}$-Arg$^{B32}$-OH
Humaninsulin-Val$^{B31}$-Arg$^{B32}$-Arg$^{B33}$-OH
Humaninsulin-Arg$^{B31}$-Argininol$^{B32}$
Humaninsulin-Lys$^{B31}$-Arg$^{B32}$-Arg$^{B33}$-OH

Humaninsulin-Arg$^{B31}$-NH- 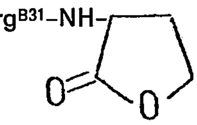

Humaninsulin-Arg$^{B31}$-Arg$^{B32}$-NH- 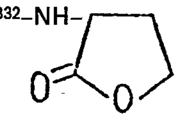

Humaninsulin-Arg$^{B31}$-$NH_2$
Humaninsulin-Arg$^{B31}$-Arg$^{B32}$-$NH_2$
Humaninsulin-Orn$^{B31}$-OH
Humaninsulin-Leu$^{B31}$-Cit$^{B32}$-OH
Humaninsulin-(B30)-$OCH_2CH_2$-$NH_2$
Humaninsulin-(B30)-NH-$CH_2CH_2$-$NH_2$
Humaninsulin-Arg$^{B31}$-O-$CH_2$-$CH_2$-$NH_2$
Humaninsulin-Arg$^{B31}$-NH-$CH_2$-$CH_2$-$N(CH_3)_2$
Humaninsulin-(B30)-O-$CH_2$-$CH_2$-$^{\oplus}N(CH_3)_3$
Humaninsulin-(B30)-NH-$CH_2$-$CH_2$-$^{\oplus}N(CH)_3$
Humaninsulin-Leu$^{B31}$-O-$CH_2$-$CH_2$-$CH_2$-$^{\oplus}N\ (C_2H_5)_3$
Humaninsulin-Trp$^{B31}$-Trp$^{B32}$-Trp$^{B33}$-$NH(CH_2)_6$-$\overset{\oplus}{N}(nC_4H_9)_3$

Die Herstellung der Insulin-Derivate der Formel I erfolgt dadurch, dass man

a) ein Des-Octapeptid (B 23-30)-Insulin der Formel II,

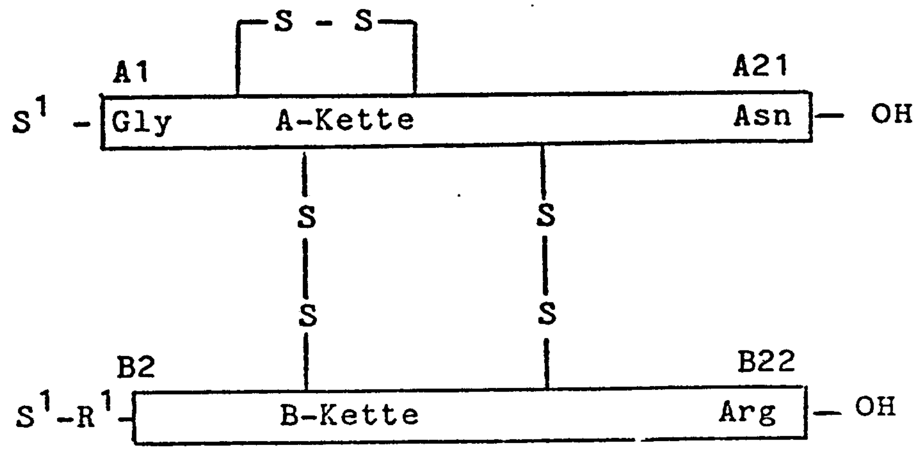

(II)

in der $R^1$ Phe oder eine Bindung und $S^1$ eine protonensolvolytisch oder durch β-Eliminierung abspaltbare Aminoschutzgruppe wie den tert-Butyloxycarbonyl-(Boc), den tert-Amyloxycarbonyl-(Aoc) oder den Methylsulfonyl-ethyloxycarbonyl-(Msc)-rest bedeuten, kondensiert mit einem Peptid der Formel III

H-Gly-Phe-Phe-Tyr($S^2$)-Thr($S^2$)-
Pro-Lys ($S^3$)-$R^{30}$-$R^{31}$ (III)

in welcher $R^{30}$ und $R^{31}$ oben definierten Bedeutungen haben, $S^2$ für Wasserstoff, Bzl (Benzyl) oder $Bu^t$ (t-Butyl) und $S^3$ für eine Urethanschutzgruppe, wie Boc (tert.-Butyloxycarbonyl) Moc (Methyloxycarbonyl) oder Z (Benzyloxycarbonyl) stehen, wobei an den Resten $R^{30}$ und $R^{31}$ gegebenenfalls vorhandene freie COOH-, OH-, SH-, -$NH_2$-, Guanidino- und/oder Imidazol-Gruppen, falls erforderlich, in an sich bekannter Weise geschützt vorliegen, und gegebenenfalls vorhandene Schutzgruppen in an sich bekannter Weise abspaltet,

b) ein Des-B30-Insulin der Formel I, in welcher $R^1$ für H oder H-Phe und der C-Terminus $R^{30}$-$R^{31}$ zusammen für OH stehen, in Gegenwart von Trypsin oder einer trypsinähnlichen endo-Peptidase umsetzt mit einer Verbindung der Formel IV

H-$R^{30}$-$R^{31}$ (IV)

in der $R^{30}$ und $R^{31}$ die oben definierten Bedeutungen haben und worin vorhandene freie COOH-, OH-, SH, W-$NH_2$-, Guanidino und/oder Imidazol-Funktionen, falls erforderlich, in an sich bekannter Weise geschützt vorliegen und anschliessend gegebenenfalls vorhandene Schutzgruppen in an sich bekannter Weise abspaltet oder

c) zur Herstellung eines Insulin-Derivats mit L-konfigurierten Aminosäureresten im Rest $R^{31}$ ein Proinsulin, Proinsulinanaloges oder Präproinsulinanaloges oder ein Intermediat dieser Verbindungen chemisch und/oder enzymatisch spaltet.

Die bei der Verfahrensvariante b) als Ausgangsverbindungen verwendeten Des-B30-Insuline sind beispielsweise aus der EP-A 46 979 oder Hoope-Seyler's Z. Physiol. Chem. 359 (1978) 799 bekannt. Dass bei Variante b) verwendete Ausgangsmaterial der Formel IV wird in an sich bekannter Weise nach den Methoden der Peptidchemie hergestellt. Brauchbare Schutzgruppen für IV sind detailliert beschrieben bei M. Bodanzky et al., Peptide Synthesis, 2nd Ed. 1976, Wiley & Sons.

Durch gentechnologische Methoden sind inzwischen humanes oder Primaten-Proinsulin als Ausgangsmaterial für die Verfahrensvariante c) zugänglich: Die Derivate Arg(B31) und Di-Arg(B31-32) sind daraus durch einfache Verdauung mit Trypsin oder trypsin-ähnlichen Enzymen zugänglich. Daneben lassen sich aber auch relativ einfach Plasmide konstruieren, die zu neuen Insulinderivaten führen, weil sie an Stelle der natürlich an B31 oder B32 befindlichen Arginine andere neutrale oder basische Aminosäuren codieren.

Die Herstellung von Proinsulin unter Anwendung der Rekombinations-DNA-Methodologie erfordert die Bildung einer DNA-Sequenz die für die Aminosäuresequenz eines Proinsulins codiert, was sich entweder durch Isolierung, Konstruktion oder eine Kombination aus beidem erreichen lässt. Die Proinsulin-DNA wird dann in Lesephase in einen geeigneten Clonierungs- und Expressionsträger eingesetzt. Der Träger dient zur Transformierung eines geeigneten Mikroorganismus, und der hierbei erhaltene transformierte Mikroorganismus wird dann Fermentationsbedingungen unterzogen, die zur Bildung weiterer Kopien des proinsulinhaltigen Vektors und zur Expression von Proinsulin eines Proinsulinderivats oder einem Proinsulinvorläufer bzw. einem Präproinsulinderivat führen.

Handelt es sich beim Expressionsprodukt um einen Proinsulinvorläufer, dann enthält ein solches Produkt im allgemeinen die Proinsulinaminosäuresequenz, die an ihrer endständigen Aminogruppe an ein Bruchstück eines Proteins gebunden ist, das normalerweise durch die Gensequenz ausgedrückt wird, bei welcher das Proinsulin oder Proinsulinderivat eingesetzt worden ist. Die Proinsulinaminosäuresequenz ist an das Proteinbruchstück über eine spezifisch spaltbare

Stelle gebunden, bei der es sich beispielsweise um Methionin handelt.

Die erhaltene Proinsulinaminosäuresequenz wird vom verschmolzenen Genprodukt, beispielsweise wie in der De-A 3 232 036 beschrieben, abgespalten und das Proinsulin nach Reinigung isoliert.

Die enzymatische Spaltung des auf diese Weise erhaltenen Proinsulins oder Proinsulinderivats erfolgt in Analogie zu der in Excerpta Medica International Congress Series No. 231, Seite 292 ff. oder der in der De-A 3 209 184 beschriebenen Verfahrensweise.

Mit Hilfe der beschriebenen semisynthetischen Verfahren sind damit zusätzlich zu den bekannten Arginin(B30)- und Diarginin(B31-32)-Derivaten sowie jenen durch gentechnologischen Methoden zugänglichen Derivaten, die an $R^{31}$natürliche L-Aminosäuren tragen, eine Reihe von neuen Insulin-Derivaten zugänglich, die als Charakteristikum eine oder mehrere basische Gruppen und/oder die Abwesenheit der freien Carboxylgruppe aufweisen, so dass die Nettoladung des Moleküls um mindestens eine positive Ladung gegenüber nativem Insulin oder gegenüber Des-$Phe^{B1}$-Insulin zunimmt.

Dazu gehören z.B. Derivate, die an Position B-31 statt der natürlichen Aminosäuren L-Lysin, L-Histidin oder L-Arginin, deren D-Enantiomere oder gängige D- oder L-Aminosäureanaloge, die in der Seitenkette eine basische Gruppierung (z.B. Ornithin, Hydroxylysin) tragen, aufweisen. Statt einer Aminosäure kann an Stelle der Position B31 beispielsweise die Cholinestergruppe treten, wodurch netto zwei Positivladungen gewonnen werden. Die Mainosäure oder das Aminosäureanalogon an Position B31 kann ein freies Carboxylende aufweisen oder mit einfachen Alkoholen (z.B. Methanol, Ethanol) verestert bzw. mit einfachen Stickstoffbasen (z.B. Ammoniak, Mono- oder Di-methylamin) amidiert sein; sie kann daneben z.B. mit Cholin verestert sein. An Position B31 kann beispielsweise eine neutrale oder auch eine weitere natürliche basische Aminosäure oder eines der oben beschriebenen Aminosäurederivate folgen; in analoger Weise kann deren Carboxylgruppe frei oder verestert bzw. amidiert sein. Es kann beispielsweise auch hier die Cholinestergruppe bzw. noch eine weitere neutrale oder basische Aminosäure bzw. ein Aminosäureanalogon folgen.

Allen diesen Insulinderivaten ist gemeinsam, dass die zusätzliche(n), an der Oberfläche des Moleküls befindliche(n) positive(n) Ladung(en) dem Molekül einen in den Neutralbereich hinein verschobenen isoelektrischen Punkt verleihen. Je nach Derivat werden isoelektrische Punkte von 5,8 bis 8,5, insbesondere 6,2 bis 8,2 in der isoelektrischen Fokussierung gemessen. Damit sind die Derivate im Neutralbereich weniger löslich als natives Insulin oedr Proinsulin, die ihren isoelektrischen Punkt und damit den Bereich maximaler Unlöslichkeit bei pH = 5,4 haben, während sie im Neutralbereich normalerweise gelöst vorliegen.

Demzufolge stellen diese Insulin-Derivate der Formel I vollkommen neue Verzögerungsprinzipien dar, die ohne Depot-Hilfsstoffe, wie Zink oder Protaminsulfat, zur Wirkung gebracht werden können. Die Depotwirkung geht auf ein inhärentes, proteinchemisch bedingtes, physikalisches Prinzip, die Schwerlöslichkeit am isoelektrischen Punkt, zurück. Die Wiederauflösung unter physiologischen Bedingungen sollte, wie man annehmen wird, durch Abspaltung der zusätzlichen basischen Gruppen erreicht werden, die je nach Derivat durch tryptische oder trypsin-ähnliche, und/oder Carboxypeptidase B- oder Carboxypeptidase B-ähnliche und/oder Esterase-Aktivität zustande kommt. Die jeweils abgespaltenen Gruppen sind entweder rein physiologische Metaboliten, wie Aminosäuren, Ornithin oder Cholin, oder aber physiologisch unbedenkliche und leicht metabolisierbare Substanzen.

Schweineinsulin-$Arg^{B31}$-OH und das entsprechende Diarginin-Derivat weisen den Untersuchungen von Chance, Excerpta Medica International Congress Series No. 231, Seite 292, 293 zufolge nur 62% bzw. 66% der Aktivität des nativen Schweineinsulins auf.

Überraschenderweise wurde nun gefunden, dass (auch im Unterschied zu Proinsulin) die biologische Aktivität der Derivate etwa gleich hoch ist wie die von nativem Insulin. Sie sind auch im Unterschied zu der in der Literatur beschriebenen Intermediaten, die noch Teile des heterologen C-Peptids enthalten, nicht stärker immunogen als das entsprechende Insulin selbst. Möglicherweise haben die oben genannten, zu niedrigen Werte von Chance ihre Ursache darin, dass diese Peptide nicht rein vorgelegen haben oder dass die Messung mit einem systematischen Fehler behaftet war.

Neben der Anwendung der beschriebenen Derivate allein oder in Mischung als reine Verzögerungsinsuline oder in Kombination mit den bekannten Depotträgern ist es nun möglich, in vielfältiger Weise stabile Mischungen mit schnell zur Verfügung stehendem Insulin, z.B. gelösten Anteilen, herzustellen. Es ist damit eine Palette von sehr fein abgestimmten Wirkprofilen zugänglich.

Besonders in Frage kommen neutrale Mischungen aus einem oder mehreren Derivaten, die als Retard-Komponente wirken, mit gelöstem, nativem Insulin, bevorzugt der gleichen Spezies. Daneben können aber auch Proinsulin und/oder C-Peptid, jeweils allein oder in Kombination mit Insulin als gelöste Komponente eingesetzt werden. Charakteristisch für diese Zubereitungen ist, dass sie in jedem Mischungsverhältnis stabil sind. Dies ist Voraussetzung für die Herstellung der heute in der Therapie sehr gebräuchlichen, intermediär wirksamen Insulinpräparate.

Die erfindungsgemässen Mittel können auch mehrere verschiedene Insulin-Derivate der Formel I und/oder mehrere verschiedene Insuline der Formel I enthalten. Darüberhinaus können auch andere therapeutisch interessante Kombinationen zur Anwendung kommen, wie etwa die Mischung von Derivat und Insulin und/oder Proinsulin und/oder Des-$Phe^{B1}$-Insulin und/oder

C-Peptid in gelöster Form oder in Form von NPH-Kristallen oder anderen klassischen Verzögerungsformen. Auf diese Weise lassen sich u.a. sehr lang wirksame Präparate mit differenziertem Basalprofil herstellen. Gerade beim Humaninsulin wäre dies wünschenswert, da nach den bisherigen Erfahrungen dessen Wirkungsdauer weder in Form der Zinkkristalle noch in Form der NPH-Kristalle ein echtes Ultra-Retard-Profil, wie etwa die analogen Rinderinsulinpräparate, aufweist.

Insulin und/oder Proinsulin und/oder Des-$Phe^{B1}$-Insulin und/oder C-Peptid und Insulin-Derivat der Formel I können auch in Form eines Alkalisalzes oder des Ammoniumsalzes eingesetzt werden.

Die Mischungsverhältnisse zwischen nativem Insulin und/oder Proinsulin und/oder Des-$Phe^{B1}$-Insulin und/oder C-Peptid und Insulinderivat können sich im Bereich 0 bis 99 Prozent Insulin und 0 bis 99 Prozent Proinsulin und 0–99 Prozent des Phenylalanin-(B1)-Insulin und 0–99 Prozent C-Peptid und 1 bis 100 Prozent Insulinderivat (bezogen auf die Gesamtmenge an diesen Peptiden) bewegen.

Eine erfindungsgemässe Anwendungsform ist auch eine saure Lösung z.B. von Insulin-Derivat und Insulin oder Proinsulin, die einen pH unterhalb des isoelektrischen Punktes des Insulins aufweist.

Bevorzugte Mittel weisen einen pH-Wert zwischen 2,5 und 8,5 auf und liegen dabei als Lösung oder Suspension vor.

Typischerweise werden die beschriebenen Anwendungsformen in einem wässrigen Medium gelöst bzw. suspendiert sein, welches zusätzlich ein geeignetes Isotoniemittel, z.B. Glycerin oder Kochsalz und ein geeignetes Mittel gegen mikrobiellen Befall, z.B. Phenol, m-Kresol oder p-Hydroxybenzoesäureester, in geeigneter Dosierung enthält.

Dieser physiologisch unbedenkliche Träger kann im pH-Bereich von 5,0 und 8,5 zusätzlich eine Puffersubstanz, z.B. Natriumacetat, Natriumcitrat, Natriumphosphat oder Tris-(hydroxymethyl)-aminomethan, enthalten. Zum Lösen und zum Einstellen des pH-Wertes werden verdünnte Säuren, typischerweise Salzsäure, oder verdünnte Laugen, typischerweise Natronlauge, verwendet.

Insulinanteil und/oder Proinsulinanteil und/oder des$Phe^{B1}$-Insulinanteil und/oder C-Peptidanteil können unabhängig voneinander jeweils in gelöster, amorpher und/oder kristalliner Form vorliegen. Jeweils ein beliebiger Teil des Insulinanteils und/oder Proinsulinanteils und/oder desPhe-Insulinanteils und/oder C-Peptidanteils und des Anteils des Insulin-Derivates der Formel I kann in kristalliner Form, jeweils ein weiterer beliebiger Teil des Insulinanteils und/oder Proinsulinanteils und/oder desPhe-Insulinanteils und/oder C-Peptidanteils und des Anteils des Insulin-Derivats der Formel I in amorpher Form sowie jeweils der Rest des Insulinanteils und/oder Proinsulinanteils und/oder des Phe-Insulinanteils und/oder C-Peptidanteils und des Anteils des Insulin-Derivats der Formel I in gelöster Form vorliegen.

Die Zubereitung kann in geeigneten Mengen Hilfsmittel mit verzögernder Wirkung (Depot-Hilfsmittel) wie etwa Protaminsulfat, Globin oder Zink (0 bis 100 µg/100 I.U.) enthalten.

Dieses Verzögerungsprinzip kann in Kombination mit dem gesamten Wirkstoffanteil oder Teilen davon angewandt werden. Auch können mehrere verschiedene verzögernd wirkende Hilfsstoffe enthalten sein.

Es ist manchmal vorteilhaft, der erfindungsgemässen Zubereitung eine geeignete Menge eines geeigneten Stabilisators zuzusetzen, der die Präzipitation von Protein bei thermisch-mechanischer Belastung bei Kontakt mit verschiedenen Materialien verhindert. Solche Stabilisatoren sind beispielsweise aus der EP-A 18 609 der DE-A 3 240 177 oder aus der WO-83/00 288 bekannt.

Zur weiteren Erläuterung der Erfindung sollen die folgenden Beispiele dienen, ohne die Erfindung auf diese zu beschränken.

Beispiel 1

Insulin-$Arg^{B31}$-$Arg^{B32}$-OH vom Schwein, hergestellt durch tryptische Verdauung aus Schweine-Proinsulin in neutraler Formulierung mit 40 IE/ml und dessen Mischbarkeit mit 20% bzw. 40% gelöstem Schweineinsulin (40 IE/ml):

Man löst in einem Gesamt-Volumen von 10 ml mit Wasser:

| | |
|---|---|
| Insulin-$Arg^{B31}$-$Arg^{B32}$-OH vom Schwein (27,0 IE/mg) | 14,8 mg |
| Natriumdihydrogenphosphat-dihydrat | 21,0 mg |
| Glycerin | 160,0 mg |
| Phenol | 6,0 mg |
| m-Kresol | 15,0 mg |

Der pH wird durch Zugabe von 1 N HCl bzw. 1 N NaOH auf pH = 7,3 eingestellt. Eine Lösung von Schweineinsulin von 40 IE/ml in einem ähnlichen oder dem gleichen Medium wird zugemischt, so dass dessen volumenmässiger Anteil 20% bzw. 40% beträgt. Mit Hilfe der HPLC wird jeweils sofort und nach 3 Monaten Lagerung bei 4°C der Gesamt-Gehalt sowie der Gehalt im Überstand ermittelt.

| | Gesamt-Bestimmung | | Überstand | |
|---|---|---|---|---|
| | t = 0 | 3 Monate 4°C | t = 0 | 3 Monate 4°C |
| 20% | 40 IE/ml | 40 IE/ml | 7,5 IE/ml | 7,8 IE/ml |
| 40% | 40 IE/ml | 40 IE/ml | 16,2 IE/ml | 15,6 IE/ml |

Schweineinsulin-$Arg^{B31}$-$Arg^{B32}$-OH trennt sich auf der HPLC von Schweineinsulin ab. Im Überstand ist kein Derivat nachweisbar, d.h. der unlösliche Anteil wird nicht aufgelöst. Umgekehrt ist nach Waschen des Niederschlages kein Insulin nachweisbar, d.h. Insulin fällt auch nicht aus.

Beispiel 2

Schweineinsulin-$Arg^{B31}$-OH, hergestellt durch tryptische Verdauung aus Schweine-Proinsulin, in Mischung mit 25% (Aktivität) gelöstem Schweineproinsulin in neutraler Formulierung mit 40 IE/ml und dessen Depot-Wirkung:

Man mischt in einem Gesamt-Volumen von 10 ml mit Wasser:

| | |
|---|---|
| Schweineinsulin-$Arg^{B31}$-OH 27,5 IE/mg | 10,9 mg |
| Schweineproinsulin 3,3 IE/mg | 30,3 mg |
| Natriumacetat | 14,0 mg |
| p-Hydroxybenzoesäuremethylester | 10,0 mg |
| Kochsalz | 80,0 mg |

Durch Zugabe von 1 N HCl bzw. 1 N NaOH wird pH = 7,0 eingestellt.

Eine solche Suspension zeigt am Hund eine Depotwirkung, die ähnlich einem Depot-Vergleichspräparat (Optisulin (R) Depot CS) ist.

Beispiel 3

Schweineinsulin-$Arg^{B31}$-$Arg^{B32}$-OH, hergestellt aus Schweineproinsulin durch tryptische Verdauung, in Form von NPH-Kristallen, in Mischung mit 25% des-Phenylalanin-(B1)-Schweineinsulin, hergestellt aus Schweineinsulin durch Edmann Abbau, in neutraler Formulierung mit 40 IE/ml und deren verzögerte Wirkung.

Man mischt in einem Gesamt-Volumen von 10 ml mit Wasser:

| | |
|---|---|
| Schweineinsulin-$Arg^{B31}$-$Arg^{B32}$-OH 27,0 IE/mg | 11,1 mg |
| des-Phenylalanin-(B1)-Schweineinsulin 28,0 IE/mg | 3,6 mg |
| Protaminsulfat | 1,0 mg |
| Natriumdihydrogenphosphat-dihydrat | 21,0 mg |
| Phenol | 6,0 mg |
| m-Kresol | 16,0 mg |
| Glycerin | 160,0 mg |

Durch Zugabe von 1 N HCl bzw. 1 N NaOH wird pH = 7,3 eingestellt.

Eine solche Suspension zeigt am Hund einen depotähnlichen Wirkungsverlauf.

Beispiel 4

Humaninsulin-(B30)-Cholinester, hergestellt aus Schweineinsulin durch Semisynthese, in Mischung mit 40% Humaninsulin und 20% (Masse) Human-C-Peptid, in neutraler Zubereitung mit 40 IE/ml und dessen mittellange Wirkungscharakteristik:

Man mischt in einem Gesamt-Volumen von 10 ml mit Wasser:

| | |
|---|---|
| Humaninsulin (B30)-Cholinester (28 IE/mg) | 7,2 mg |
| Humaninsulin (28 IE/mg) | 7,2 mg |
| Human-C-Peptid | 3,6 mg |
| Natriumdihydrogenphosphat-dihydrat | 21,0 mg |
| m-Kresol | 27,0 mg |
| Glycerin | 160,0 mg |

Der pH-Wert wird durch Zugabe von 1 N HCl bzw. 1 N NaOH auf pH = 7,3 eingestellt.

Eine solche Suspension zeigt am Hund ein Wirkprofil vergleichbar einem Kombinationspräparat (z.B. Komb-H-Insulin(R) Hoechst).

Beispiel 5

Humaninsulin-$Arg^{B31}$-$Lys^{B32}$-$OCH_3$, hergestellt durch Semisynthese aus Schweineinsulin, in Mischung mit 50% Zink-Humaninsulinkristallen in einer Zubereitung mit 40 IE/ml und deren verzögerte Wirkung.

Man mischt in einem Gesamt-Volumen von 10 ml mit Wasser:

| | |
|---|---|
| Humaninsulin-$Arg^{B31}$-$Lys^{B32}$-$OCH_3$ (27,0 IE/mg) | 7,4 mg |
| Humaninsulin (28 IE/mg) | 7,4 mg |
| Zinkchlorid, wasserfrei | 0,23 mg |
| Natriumacetat | 14,0 mg |
| p-Hydroxybenzoesäuremethylester | 10,0 mg |
| Kochsalz | 80,0 mg |

Durch Zugabe von 1 N HCl bzw. 1 N NaOH wird pH = 7,0 eingestellt.

Eine solche Präparation hat am Kaninchen (0,4 IE/kg) eine ausgeprägte Retardwirkung.

Beispiel 6

Humaninsulin-$Arg^{B31}$-OH in Mischung mit 30% Humaninsulin-$Arg^{B31}$-$Arg^{B32}$-OH, beide hergestellt durch tryptische Verdauung aus Primaten-Präproinsulin exprimiert in E.coli, in Mischung mit 40% kristallinem NPH-Humaninsulin in einer Zubereitung mit 40IE/ml und deren ausgeprägte Retardwirkung.

Man mischt in einem Gesamt-Volumen von 10 ml mit Wasser:

| | |
|---|---|
| Humaninsulin-$Arg^{B31}$-OH (27,5 IE/mg) | 4,4 mg |
| Humaninsulin-$Arg^{B31}$-$Arg^{B32}$-OH (27,0 IE/mg) | 4,4 mg |
| Humaninsulin (28 IE/mg) | 5,7 mg |
| Protaminsulfat | 0,5 mg |
| Natriumdihydrogenphosphat-dihydrat | 21,0 mg |
| m-Kresol | 15,0 mg |
| Phenol | 6,0 mg |
| Glycerin | 160,0 mg |

Durch Zugabe von 1 N NaOH bzw. 1 N HCl wird pH = 7,3 eingestellt.

Eine solche Suspension zeigt am Kaninchen eine stark verzögerte und lang anhaltende Wirkung.

**Patentansprüche für die Vertragsstaaten BE CH DE FR GB IT LI LU NL SE**

1. Arzneimittel aus einem physiologisch unbedenklichen Träger und einer Wirkstoffkombination, dadurch gekennzeichnet, dass es als Wirkstoffkombination

a) ein Insulin-Derivat der Formel I,

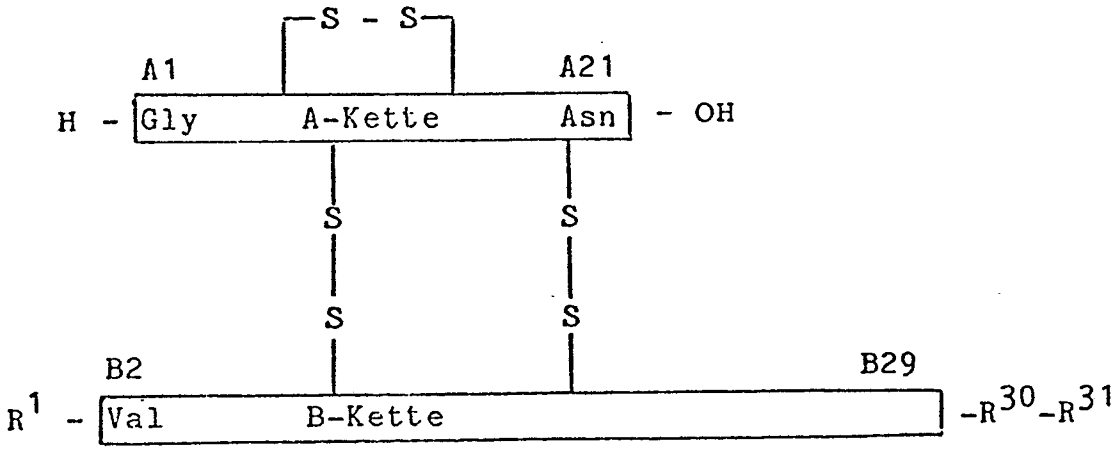

(I)

in welcher
$R^1$ H oder H-Phe bedeutet,
$R^{30}$ für den Rest einer neutralen, genetisch kodierbaren L-Aminosäure steht und
$R^{31}$ für -OH oder eine physiologisch unbedenkliche organische Gruppe basischen Charakters mit bis zu 50 C-Atomen steht, an deren Aufbau 0 bis 3 α-Aminosäuren beteiligt sind und deren gegebenenfalls vorhandene endständige Carboxyfunktion frei, als Esterfunktion, als Amidfunktion, als Lacton oder zu $CH_2OH$ reduziert vorliegen kann,

mit einem isoelektrischen Punkt zwischen 5,8 und 8,5, und
$b_1$) ein Insulin der Formel I, in welcher $R^1$ H oder H-Phe bedeutet, $R^{30}$ für Ala, Thr oder Ser steht und $R^{31}$ OH bedeutet, oder
$b_2$) physiologisch unbedenkliche Salze davon, oder
$b_3$) Proinsulin oder
$b_4$) C-Peptid oder
$b_5$) eine Kombination von $b_3$) und $b_4$) enthält,

wobei Komponente a) in einer Menge von mindestens 1% zugegen ist.

2. Mittel gemäss Anspruch 1, dadurch gekennzeichnet, dass in dem unter a) genannten Insulin-Derivat der Formel I
$R^{31}$ für den Rest der Formel $-X_n-S$ steht,
in welchem
n = 0, 1, 2 oder 3 ist,
X für gleiche oder verschiedene Reste natürlich vorkommender neutraler oder basischer L-Aminosäuren und/oder der diesen entsprechenden D-Aminosäuren steht und
S OH oder eine physiologisch unbedenkliche, die Carboxygruppe blockierende Gruppe bedeutet, die, falls n = 0 ist, einen positiv geladenen oder protonierbaren basischen Rest trägt oder, falls n > 0 ist, einen solchen Rest tragen kann und worin der C-Terminus
-X-S auch für den Rest einer zum entsprechenden Alkohol reduzierten Aminosäure oder, im Falle n = 2 oder 3, für den Homoserinlacton-Rest stehen kann.

3. Mittel gemäss Anspruch 1 oder 2, dadurch gekennzeichnet, dass im Insulin-Derivat und im Insulin der Formel I $R^1$ für H-Phe steht.

4. Mittel gemäss einem der Ansprüche 1 bis 3, dadurch gekennzeichnet, dass in dem unter 1 a) genannten Insulin-Derivat der Formel I $R^{30}$ für Ala, Thr oder Ser steht.

5. Mittel gemäss einem der Ansprüche 2 bis 4, dadurch gekennzeichnet, dass in dem unter 1a) genannten Insulin-Derivat der Formel I die Aminosäurereste X in der L-Konfiguration vorliegen.

6. Mittel gemäss einem der Ansprüche 2 bis 5, dadurch gekennzeichnet, dass in dem unter 1a) genannten Insulin-Derivat der Formel I S für OH, ($C_1$ bis $C_6$)-Alkoxy, ($C_3$ bis $C_6$)-Cycloalkyloxy, $NH_2$, Di-($C_1$ bis $C_6$)-alkylamino, ($C_1$ bis $C_6$)-Alkylamino, Amino-($C_2$ bis $C_6$)-alkoxy, ($C_2$ bis $C_4$)-Alkylamino-($C_2$ bis $C_6$)-alkoxy, Di($C_1$ bis $C_4$)-alkylamino-($C_2$ bis $C_6$)-alkoxy, Tri($C_1$ bis $C_4$)-alkyl-ammonic-($C_2$ bis $C_6$)-alkoxy, Amino-($C_2$ bis $C_6$)-alkylamin, [($C_1$ bis $C_4$)-alkylamino]-($C_2$ bis $C_6$)-alkylamino, [Di($C_1$ bis $C_4$)-alkylamino]-($C_2$ bis $C_6$)-alkylamino oder [Tri-($C_1$ bis $C_4$)-alkylammonio]-($C_2$ bis $C_6$)-alkylamino steht.

7. Mittel gemäss einem der Ansprüche 2 bis 6, dadurch gekennzeichnet, dass in dem unter 1a) genannten Insulin-Derivat der Formel I X einen Rest einer natürlich vorkommenden basischen Aminosäure, wie Lys, Arg, His, Cit, Orn oder Hyl und/oder deren D-Formen bedeutet.

8. Mittel gemäss einem der Ansprüche 1 bis 7, dadurch gekennzeichnet, dass es Insulin-B 31-Arg-OH oder Insulin-B 31-Arg-Arg-OH enthält.

9. Mittel gemäss einem der Ansprüche 1 bis 8, dadurch gekennzeichnet, dass es mehrere verschiedene Insulin-Derivate der Formel I und/oder

mehrere verschiedene Insuline der Formel I enthält.

10. Mittel gemäss einem der Ansprüche 1 bis 9, dadurch gekennzeichnet, dass es ein Proinsulin oder Proinsulinanaloges und/oder humanes C-Peptid enthält.

11. Mittel gemäss einem der Ansprüche 1 bis 10, dadurch gekennzeichnet, dass es humanes C-Peptid enthält.

12. Mittel gemäss einem der Ansprüche 1 bis 11, dadurch gekennzeichnet, dass dieses einen pH zwischen 2,5 und 8,5 aufweist, und dabei als Lösung oder Suspension vorliegt und ein geeignetes Isotonie-Mittel in der üblichen Menge sowie ein geeignetes Konservierungsmittel in geeigneter Menge enthält.

13. Mittel gemäss einem der Ansprüche 1 bis 12, dadurch gekennzeichnet, dass dieses zusätzlich eine geeignete Menge einer geeigneten Puffersubstanz enthält, wenn der pH-Wert zwischen 5,0 und 8,5 liegt.

14. Mittel gemäss einem der Ansprüche 1 bis 13, dadurch gekennzeichnet, dass die Zubereitung eine geeignete Menge eines geeigneten Stabilisators enthält, der die Präzipitation von Protein bei thermisch-mechanischer Belastung bei Kontakt mit verschiedenen Materialien verhindert.

13. Mittel gemäss einem der Ansprüche 1 bis 14, dadurch gekennzeichnet, dass dieses eine geeignete Menge Zink enthält, die zwischen 0 und 100 µg/100 Einheiten liegen kann.

16. Mittel gemäss einem der Ansprüche 1 bis 15, dadurch gekennzeichnet, dass Insulin und/oder Proinsulin und/oder $desPhe^{B1}$-Insulin und/oder C-Peptid und Insulin-Derivat der Formel I in Form eines Alkalisalzes oder des Ammoniumsalzes eingesetzt werden.

17. Mittel gemäss einem der Ansprüche 1 bis 16, dadurch gekennzeichnet, dass Insulinanteil und/oder Proinsulinanteil und/oder $desPhe^{B1}$-Insulinanteil und/oder C-Peptidanteil und Anteil des Insulin-Derivats der Formel I unabhängig voneinander jeweils in gelöster amorpher und/oder kristalliner Form vorliegen können.

18. Mittel gemäss einem der Ansprüche 1 bis 17, dadurch gekennzeichnet, dass jeweils ein beliebiger Teil des Insulinanteils und/oder Proinsulinanteils und/oder desPhe-Insulinanteils und/oder C-Peptidanteils und Anteil des Insulin-Derivats der Formel I in kristalliner Form, jeweils ein weiterer beliebiger Teil des Insulinanteils und/oder Proinsulinanteils und/oder desPhe-Insulinanteils und/oder C-Peptidanteils und des Anteils des Insulin-Derivats der Formel I in amorpher Form sowie jeweils der Rest des Insulinanteils und/oder Proinsulin und/oder desPhe-Insulinanteils und/oder C-Peptidanteils und des Anteils des Insulin-Derivats der Formel I in gelöster Form vorliegt.

19. Mittel gemäss einem der Ansprüche 1 bis 18, dadurch gekennzeichnet, dass die Zubereitung eines der bekannten Hilfsmittel mit verzögernder Wirkung in geeigneten Mengen, enthält.

20. Mittel gemäss Anspruch 19, dadurch gekennzeichnet, dass dieses Verzögerungsprinzip in Kombination mit dem gesamten Wirkstoffanteil oder mit Teilen davon angewandt wird.

21. Mittel gemäss einem der Ansprüche 19 oder 20, dadurch gekennzeichnet, dass es Insulin und/oder Proinsulin und/oder desPhe-Insulin und/oder C-Peptid und Insulin-Derivat der Formel I in Kombination mit mehreren verschiedenen verzögernd wirkenden Hilfsstoffen enthält.

22. Verfahren zur Herstellung eines Mittels gemäss einem der Ansprüche 1 bis 21, dadurch gekennzeichnet, dass man die Wirkstoffkombination in eine geeignete Darreichungsform bringt.

23. Arzneimittel gemäss einem der Ansprüche 1 bis 21 zur Verwendung bei der Behandlung des Diabetes mellitus.

**Patentansprüche für den Vertragsstaat AT**

1. Verfahren zur Herstellung eines Arzneimittels aus einem physiologisch unbedenklichen Träger und einer Wirkstoffkombination aus

a) einem Insulin-Derivat der Fromel I

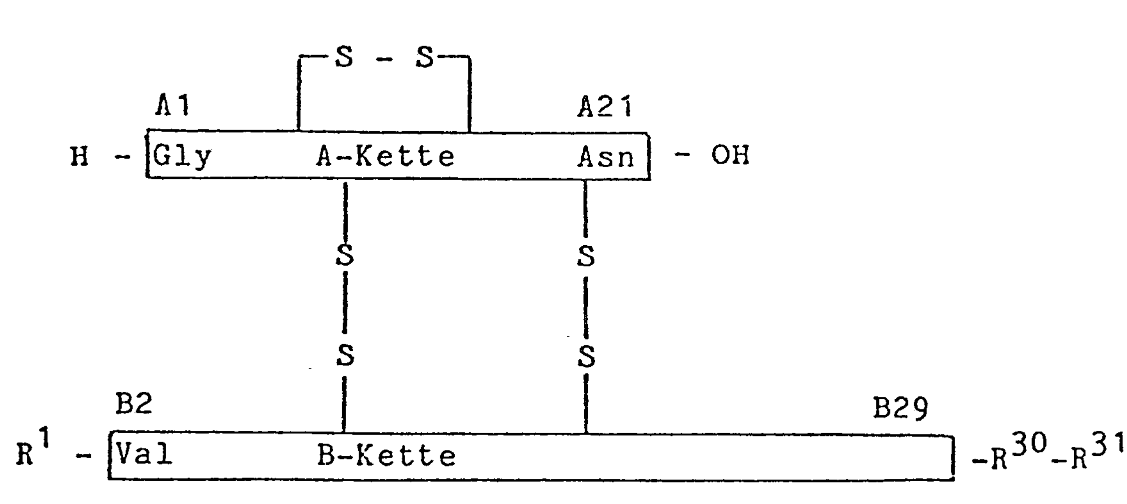

(I)

in welcher
$R^1$ H oder H-Phe bedeutet,
$R^{30}$ für den Rest einer neutralen, genetisch kodierbaren L-Aminosäure steht und
$R^{31}$ für -OH oder eine physiologisch unbedenkliche organische Gruppe basischen Charakters mit bis zu 50 C-Atomen steht, an deren Aufbau 0 bis 3 α-Aminosäuren beteiligt sind und deren gegebenenfalls vorhandene endständige Carboxyfunktion frei, als Esterfunktion, als Amidfunktion, als Lacton oder zu $CH_2OH$ reduziert vorliegen kann, mit einem isoelektrischen Punkt zwischen 5,8 und 8,5 und
$b_1$) ein Insulin der Formel I, in welcher $R^1$H oder H-Phe bedeutet, $R^{30}$ für Ala, Thr oder Ser steht und $R^{31}$ OH bedeutet, oder
$b_2$) physiologisch unbedenkliche Salze davon, oder
$b_3$) Proinsulin oder
$b_4$) C-Peptid oder
$b_5$) eine Kombination von $b_3$) und $b_4$) enthält,

wobei Komponente a) in einer Menge von mindestens 1% zugegen ist, dadurch gekennzeichnet, dass man diese in eine geeignete Darreichungsform bringt.

2. Verfahren gemäss Anspruch 1, dadurch gekennzeichnet, dass man ein Mittel herstellt, worin in dem unter a) genannten Insulin-Derivat der Formel I

$R^{31}$ für einen Rest der Formel $-X_n-S$ steht, in welchem
n = 0, 1, 2 oder 3 ist,
X für gleiche oder verschiedene Reste natürlich vorkommender neutraler oder basischer L-Aminosäuren und/oder der diesen entsprechenden D-Aminosäuren steht und
S OH oder eine physiologisch unbedenkliche, die Carboxygruppe blockierende Gruppe bedeutet, die, falls n = 0 ist, einen positiv geladenen oder protonierbaren basischen Rest trägt oder, falls n > 0 ist, einen solchen Rest tragen kann und worin der C-Terminus
-X-S auch für den Rest einer zum entsprechenden Alkohol reduzierten Aminosäure oder, im Falle n = 2 oder 3, für den Homoserinlacton-Rest stehen kann.

3. Verfahren gemäss Anspruch 1 oder 2, dadurch gekennzeichnet, dass man ein Mittel herstellt, worin im Insulin-Derivat und im Insulin der Formel I $R^1$ für H-Phe steht.

4. Verfahren gemäss einem der Ansprüche 1 bis 3, dadurch gekennzeichnet, dass man ein Mittel herstellt, worin in dem unter 1 a) genannten Insulin-Derivat der Formel I $R^{30}$ für Ala, Thr oder Ser steht.

5. Verfahren gemäss einem der Ansprüche 2 bis 4, dadurch gekennzeichnet, dass man ein Mittel herstellt, worin in dem unter 1 a) genannten Insulin-Derivat der Formel I die Aminosäurereste X in der L-Konfiguration vorliegen.

6. Verfahren gemäss einem der Ansprüche 2 bis 5, dadurch gekennzeichnet, dass man ein Mittel herstellt, worin in dem unter 1 a) genannten Insulin-Derivat der Formel

I S für OH, ($C_1$ bis $C_6$)-Alkoxy, ($C_3$ bis $C_6$)-Cycloalkyloxy, $NH_2$, Di-($C_1$ bis $C_6$)-alkylamino, ($C_1$ bis $C_6$)-Alkylamino, Amino-($C_2$ bis $C_6$)-alkoxy, ($C_2$ bis $C_4$)-Alkylamino-($C_2$ bis $C_6$)-alkoxy, Di($C_1$ bis $C_4$)-alkylamino-($C_2$ bis $C_6$), Tri($C_1$ bis $C_4$)ammonio-($C_2$ bis $C_6$)-alkoxy, Amino-($C_2$ bis $C_6$)-alkylamin, [($C_1$ bis $C_4$)-alkylamino]-($C_2$ bis $C_6$)-alkylamino, [Di($C_1$ bis $C_4$)-alkylamino]-($C_2$ bis $C_6$)-alkylamino oder [Tri-($C_1$ bis $C_4$)alkylammonio]-($C_2$ bis $C_6$)-alkylamine steht.

7. Verfahren gemäss einem der Ansprüche 2 bis 6, dadurch gekennzeichnet, dass man ein Mittel herstellt, worin in dem unter 1a) genannten Insulin-Derivat der Formel I und X einen Rest einer natürlich vorkommenden basischen Aminosäure, wie Lys, Arg, His, Cit, Orn oder Hyl und/oder deren D-Formen bedeutet.

8. Verfahren gemäss einem der Ansprüche 1 bis 7, dadurch gekennzeichnet, dass ein Mittel hergestellt wird, welches Insulin-B31-Arg-OH oder Insulin-B31-Arg-Arg-OH enthält.

9. Verfahren gemäss einem der Ansprüche 1 bis 8, dadurch gekennzeichnet, dass man ein Mittel herstellt, welches mehrere verschiedene Insulin-Derivate der Formel I und/oder mehrere verschiedene Insuline der Formel I enthält.

10. Verfahren gemäss einem der Ansprüche 1 bis 9, dadurch gekennzeichnet, dass man ein Mittel herstellt, welches ein Proinsulin oder Proinsulin-analoges und/oder humanes C-Peptid enthält.

11. Verfahren gemäss einem der Ansprüche 1 bis 10, dadurch gekennzeichnet, dass man ein Mittel herstellt, welches humanes C-Peptid enthält.

12. Verfahren gemäss einem der Ansprüche 1 bis 11, dadurch gekennzeichnet, dass man ein Mittel herstellt, welches einen pH zwischen 2,5 und 8,5 aufweist, und dabei als Lösung oder Suspension vorliegt und ein geeignetes Isotonie-Mittel in der üblichen Menge sowie ein geeignetes Konservierungsmittel in geeigneter Menge enthält.

13. Verfahren gemäss einem der Ansprüche 1 bis 12, dadurch gekennzeichnet, dass man ein Mittel herstellt, welches zusätzlich eine geeignete Menge einer geeigneten Puffersubstanz enthält, wenn der pH-Wert zwischen 5,0 und 8,5 liegt.

14. Verfahren gemäss einem der Ansprüche 1 bis 13, dadurch gekennzeichnet, dass man ein Mittel herstellt, welches eine geeignete Menge eines geeigneten Stabilisators enthält, der die Präzipitation von Protein bei thermisch-mechanischer Belastung bei Kontakt mit verschiedenen Materialien verhindert.

15. Verfahren gemäss einem der Ansprüche 1 bis 14, dadurch gekennzeichnet, dass man ein Mittel herstellt, welches eine geeignete Menge Zink enthält, die zwischen 0 und 100 µg/100 Einheiten liegen kann.

16. Verfahren gemäss einem der Ansprüche 1 bis 15, dadurch gekennzeichnet, dass man ein

Mittel herstellt, worin Insulin und/oder Proinsulin und/oder desPhe$^{B1}$-Insulin und/oder C-Peptid und Insulin-Derivat der Formel I in Form eines Alkalisalzes oder des Ammoniumsalzes eingesetzt werden.

17. Verfahren gemäss einem der Ansprüche 1 bis 16, dadurch gekennzeichnet, dass man ein Mittel herstellt, worin Insulinanteil und/oder Proinsulinanteil und/oder desPhe$^{B1}$-Insulinanteil und/oder C-Peptidanteil und Anteil des Insulin-Derivats der Formel I unabhängig voneinander jeweils in gelöster, amorpher und/oder kristalliner Form vorliegen können.

18. Verfahren gemäss einem der Ansprüche 1 bis 17, dadurch gekennzeichnet, dass man ein Mittel herstellt, worin jeweils ein beliebiger Teil des Insulinanteils und/oder Proinsulinanteils und/oder desPhe-Insulinanteils und/oder C-Peptidanteils und Anteil des Insulin-Derivats der Formel I in kristalliner Form, jeweils ein weiterer beliebiger Teil des Insulinanteils und/oder Proinsulinanteils und/oder desPhe-Insulinanteils und/oder C-Peptidanteil und des Anteils des Insulin-Derivats der Formel I in amorpher Form sowie jeweils der Rest des Insulinanteils und/oder Proinsulin und/oder desPhe-Insulinanteils und/oder C-Peptidanteils und des Anteils des Insulin-Derivats der Formel I in gelöster Form vorliegt.

19. Verfahren gemäss einem der Ansprüche 1 bis 18, dadurch gekennzeichnet, dass man ein Mittel herstellt, welches eines der bekannten Hilfsmittel mit verzögernder Wirkung in geeigneten Mengen enthält.

20. Verfahren gemäss Anspruch 5, dadurch gekennzeichnet, dass dieses Verzögerungsprinzip in Kombination dem dem gesamten Wirkstoffanteil oder mit Teilen davon angewandt wird.

21. Verfahren gemäss einem der Ansprüche 19 oder 20, dadurch gekennzeichnet, dass man ein Mittel herstellt, welches Insulin und/oder Proinsulin und/oder desPhe-Insulin und/oder C-Peptid und Insulin-Derivat der Formel I in Kombination mit mehreren verschiedenen verzögernd wirkenden Hilfsstoffen enthält.

**Claims for the Contracting States BE CH DE FR GB IT LI LU NL SE**

1. A medicament consisting of a physiologically acceptable excipient and an active compound combination, characterized in that it contains, as the active compound combination

a) an insulin derivative of the formula I

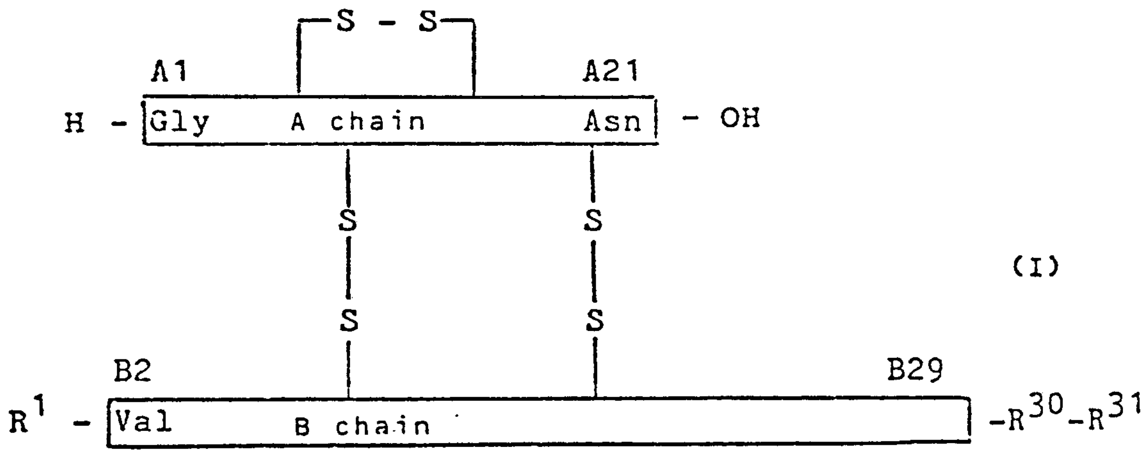

(I)

in which
$R^1$ denotes H or H-Phe,
$R^{30}$ represents the radical of a neutral L-amino-acid which can be genetically coded and
$R^{31}$ represents -OH or a physiologically acceptable organic group of basic character with up to 50 carbon atoms, in the build-up of which 0 to 3 α-aminoacids participate and in which the terminal carboxyl function optionally present can be free, as an ester function, as an amide function, as a lactone or reduced to $HC_2OH$,

with an isoelectric point between 5.8 and 8.5 and
$b_1$) an insulin of the formula I
in which
$R^1$ denotes H or H-Phe,
$R^{30}$ represents Ala, Thr or Ser and
$R^{31}$ denotes OH, or
$b_2$) physiologically acceptable salts thereof, or
$b_3$) proinsulin or
$b_4$) C-peptide or
$b_5$) a combination of $b_3$) and $b_4$),
component a being present in an amount of at least 1%.

2. An agent as claimed in claim 1, characterized in that in the insulin derivative of the formula I, mentioned under a),
$R^{31}$ represents a radical of the formula $-X_n-S$,
in which
n is 0, 1, 2 or 3,
X represents identical or different radicals of naturally occurring neutral or basic L-amino acids and/or of D-aminoacids corresponding to these, and
S denotes OH or a physiologically acceptable group which blocks the carboxyl group and which, is n is 0, carries a positively charged or

protonatable basic radical or, if n is greater than 0, can carry such a radical, and in which the C-terminus -X-S can also represent the radical of an aminoacid reduced to the corresponding alcohol, or if n is 2 or 3, can represent the homoserine-lactone radical.

3. An agent as claimed in claim 1 or 2, characterized in that $R^1$ represents H-Phe in the insulin derivative and in the insulin of the formula I.

4. An agent as claimed in any one of claims 1 to 3, characterized in that $R^{30}$ represents Ala, Thr or Ser in the insulin derivative of the formula I, mentioned under 1a).

5. An agent as claimed in any one of claims 2 to 4, characterized in that the aminoacid radicals X are in the L-configuration in the insulin derivative of the formula I, mentioned under 1a).

6. An agent as claimed in any one of claims 2 to 5, characterized in that S represents OH, ($C_1$ to $C_6$)-alkoxy, ($C_3$ to $C_6$)-cycloalkoxy, $NH_2$, di-($C_1$ to $C_6$)-alkylamino, ($C_1$ to $C_6$)-alkylamino, amino-($C_2$ to $C_6$)-alkoxy, ($C_2$ to $C_4$)-alkylamino-($C_2$ to $C_6$)-alkoxy, di-($C_1$ to $C_4$)-alkylamino-($C_2$ to $C_6$)-alkoxy, tri($C_1$ to $C_4$)-alkylammonio-($C_2$ to $C_6$)-alkoxy, amino-($C_2$ to $C_6$)-alkylamine, [($C_1$ to $C_4$)-alkylamino]-($C_2$ to $C_6$)alkylamino, [di-($C_1$ to $C_4$)-alkylamino]-($C_2$ to $C_6$)-alkylamino or [tri-($C_1$ to $C_4$)-alkylammonio]-($C_2$ to $C_6$)-alkylamino in the insulin derivative of the formula I, mentioned under 1a).

7. An agent as claimed in any one of claims 2 to 6, characterized in that X denotes a radical of a naturally occurring basic aminoacid, such as Lys, Arg, His, Cit, Orn or Hyl and/or the D-forms thereof, in the insulin derivative of the formula I, mentioned under 1a).

An agent as claimed in any one of claims 1 to 7, characterized in that it contains insulin-B31-Arg-OH or insulin-B31-Arg-Arg-OH.

9. An agent as claimed in any one of claims 1 to 8, characterized in that it contains several different insulin derivatives of the formula I and/or several different insulins of the formula I.

10. An agent as claimed in any one of claims 1 to 9, characterized in that it contains proinsulin or a proinsulin analog and/or human C peptide.

11. An agent as claimed in any one of claims 1 to 10, characterized in that it contains human C-peptide.

12. An agent as claimed in any one of claims 1 to 11, characterized in that it has a pH between 2.5 and 8.5 and is in the form of a solution or suspension, and contains a suitable isotonicity agent in the customary amount and a suitable preservative in a suitable amount.

13. An agent as claimed in any one of claims 1 to 12, characterized in that it additionally contains a suitable amount of a suitable buffer substance, if the pH value is between 5.0 and 8.5.

14. An agent as claimed in any one of claims 1 to 13, characterized in that the formulation contains a suitable amount of a suitable stabilizer which prevents precipitations of protein on exposure to heat or mechanical stress on contact with various materials.

15. An agent as claimed in any one of claims 1 to 14, characterized in that it contains a suitable amount of zinc, which can be between 0 and 100 µg/100 units.

16. An agent as claimed in any one of claims 1 to 15, characterized in that the insulin and/or proinsulin and/or des-$Phe^{B1}$-insulin and/or C-peptide and insulin derivative of the formula I are used in the form of an alkali metal salt or the ammonium salt.

17. An agent as claimed in any one of claims 1 to 16, characterized in that the insulin content and/or proinsulin content and/or des-$Phe^{B1}$-insulin content and/or C-peptide content and the content of insulin derivative of the formula I can in each case be, independently of one another, in dissolved, amorpheus and/or crystalline form.

18. An agent as claimed in any one of claims 1 to 17, characterized in that in each case any desired part of the insulin content and/or proinsulin content and/or des-Phe-insulin content and/or C-peptide content and of the content of insulin derivative of the formula I is in crystalline form, in each case any other desired part of the insulin content and/or proinsulin content and/or des-Phe-insulin content and/or C peptide content and of the content of the insulin derivative of the formula I is in amorphous form, and in each case the remainder of the insulin content and/or proinsulin content and/or des-Phe-insulin content and/or Ce peptide content and of the content of the insulin derivative of the formula I is in dissolved form.

19. An agent as claimed in any one of claims 1 to 18, characterized in that the formulation contains suitable amounts of one of the known auxiliaries having a delaying action.

20. An agent as claimed in claim 19, characterized in that this delayed action principle is used in combination with the entire active compound content or with parts thereof.

21. An agent as claimed in either of claims 19 and 20, characterized in that it contains insulin and/or proinsulin and/or des-Phe-insulin and/or C peptide and insulin derivative of the formula I in combination with several different auxiliaries having a delaying action.

22. A process for the preparation of an agent as claimed in any one of claims 1 to 21, characterized in bringing the active compound combination into a suitable form for administration.

23. A medicament as claimed in any one of claims 1 to 21 for use in the treatment of diabetes mellitus.

**Claims for the Contracting State: AT**

1. A process for the preparation of a medicament consisting of a physiologically acceptable excipient and an active compound combination, comprising

a) an insulin derivative of the formula I

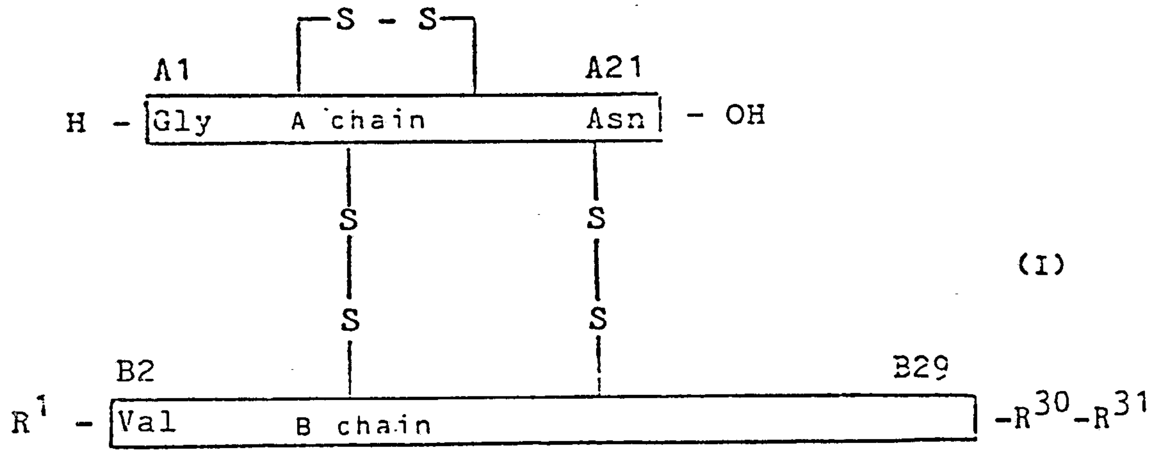

(I)

in which
$R^1$ denotes H or H-Phe,
$R^{30}$ represents the radical of a neutral L-amino-acid which can be genetically coded and
$R^{31}$ represents -OH or a physiologically acceptable organic group of basic character with up to 50 carbon atoms, in the build-up of which 0 to 3 α-aminoacids participate and in which the terminal carboxyl function optionally present can be free, as an ester function, as an amide function, as a lactone or reduced to $CH_2OH$,

with an isoelectric point between 5.8 and 8.5, and
$b_1$) an insulin of the formula I
in which
$R^1$ denotes H or H-Phe,
$R^{30}$ represents Ala,Thr or Ser and
$R^{31}$ denotes OH, or
$b_2$) physiologically acceptable salts thereof, or
$b_3$) proinsulin or
$b_4$) C-peptide or
$b_5$) a combination of $b_3$) and $b_4$),

component a being present in an amount of at least 1%, characterized in bringing said active compound combination into a suitable form for administration.

2. A process as claimed in claim 1, characterized in preparing an agent in which, in the insulin derivative of the formula I, mentioned under a)
$R^{31}$ represents a radical of the formula $-X_n-S$,
in which
n is 0, 1, 2 or 3,
X represents identical or different radicals of naturally occurring neutral or basic L-amino acids and/or of D-aminoacids corresponding to these, and
S denotes OH or a physiologically acceptable group which blocks the carboxyl group and which, if n is 0, carries a positively charged or ptotonable basic radical or, if n is greater than 0, can carry such a radical, and in which the C-terminus -X-S can also represent the radical of an aminoacid reduced to the corresponding alcohol, or if n is 2 or 3, can represent the homoserine-lactone radical.

3. A process as claimed in either of claims 1 and 2, characterized in preparing an agent, in which $R^1$ represents H-Phe in the insulin derivative and in the insulin of the formula I.

a. A process as claimed in any one of claims 1 to 3, characterized in preparing an agent, in which $R^{30}$ represents Ala, Thr or Ser in the insulin derivative of the formula I, mentioned under 1a).

5. A process as claimed in any one of claims 2 to 4, characterized in preparing an agent, in which the aminoacid radicals X are in the L-configuration in the insulin derivative of the formula I, mentioned under 1a).

6. A process as claimed in any one of claims 2 to 5, characterized in preparing an agent, in which S represents OH,
($C_1$ to $C_6$)-alkoxy, ($C_3$ to $C_6$)-cycloalkoxy, $NH_2$, di-($C_1$ to $C_6$)-alkylamino, ($C_1$ to $C_6$)-alkylamino, amino-($C_2$ to $C_6$)-alkoxy, ($C_2$ to $C_4$)-alkylamino-($C_2$ to $C_6$)-alkoxy, di-($C_1$ to $C_4$)-alkylamino-($C_2$ to $C_6$)-alkoxy, tri($C_1$ to $C_4$)-alkylammonio-($C_2$ to $C_6$)-alkoxy, amino-($C_2$ to $C_6$)-alkylamine, [($C_1$ to $C_4$)-alkylamino]-($C_2$ to $C_6$)-alkylamino, [di-($C_1$ to $C_4$)-alkylamino]-($C_2$ to $C_6$)-alkylamino or [tri-($C_1$ to $C_4$)-alkylammonio]-($C_2$ to $C_6$)-alkylamino in the insulin derivative of the formula I, mentioned under 1a).

7. A process as claimed in any one of claims 2 to 6, characterized in preparing an agent, in which X denotes a radical of a naturally occurring basic aminoacid, such as Lys, Arg, His, Cit, Orn or Hyl and/or the D-forms thereof, in the insulin derivative of the formula I, mentioned under 1a).

8. A process as claimed in any one of claims 1 to 7, characterized in preparing an agent, which contains insulin-B31-Arg-OG or insulin-B31-Arg-Arg-OH.

9. A process as claimed in any one of claims 1 to 8, characterized in preparing an agent, which contains several different insulin derivatives of the formula I and/or several different insulins of the formula I.

10. A process as claimed in any one of claims 1 to 9, characterized in preparing an agent, which contains proinsulin or a proinsulin analog and/or human C peptide.

11. A process as claimed in any one of claims 1 to 10, characterized in preparing an agent, which contains human C-peptide.

12. A process as claimed in any one of claims 1 to 11, characterized in preparing an agent, which has a pH between 2.5 and 8.5 and is in the form of a solution or suspension, and contains a suitable isotonicity agent in the customary amount and a suitable preservative in a suitable amount.

13. A process as claimed in any one of claims 1 to 12, characterized in preparing an agent, which additionally contains a suitable amount of a suitable buffer substance, if the pH value is between 5.0 and 8.5.

15. A process as claimed in any one of claims 1 to 13, characterized in preparing an agent, which contains a suitable amount of a suitable stabilizer which prevents precipitation of protein on exposure to heat or mechanical stress on contact with various materials.

15. A process as claimed in any one of claims 1 to 14, characterized in preparing an agent, which contains a suitable amount of zinc, which can be between 0 and 100 µg/100 units.

16. A process as claimed in any one of claims 1 to 15, characterized in preparing an agent, in which the insulin and/or proinsulin and/or des-$Phe^{B1}$-insulin and/or C-peptide and insulin derivative of the formula I are used in the form of an alkali metal salt or the ammonium salt.

17. A process as claimed in any one of claims 1 to 16, characterized in preparing an agent, wherein the insulin content and/or proinsulin content and/or des-$Phe^{B1}$-insulin content and/or C-peptide content and the content of insulin derivative of the formula I can in each case be, independently of one another, in dissolved, amorphous and/or crystalline form.

18. A process as claimed in any one of claims 1 to 17, characterized in preparing an agent, in which in each case any desired part of the insulin content and/or proinsulin content and/or des-Phe-insulin content and/or C-peptide content and of the content of insulin derivative of the formula I is in crystalline form, in each case any other desired part of the insulin content and/or proinsulin content and/or des-Phe-insulin content and/or C peptide content and of the content of the insulin derivative of the formula I is in amorphous form, and in each case the remainder of the insulin content and/or proinsulin content and/or des-Phe-insulin content and/or C peptide content and of the content of the insulin derivative of the formula I is in dissolved form.

19. A process as claimed in any one of claims 1 to 18, characterized in preparing an agent, which contains suitable amounts of one of the known auxiliaries having a delaying action.

20. A process as claimed in claim 19, characterized in using this delayed action principle in combination with the entire active compound content or with parts thereof.

21. A process as claimed in either of claims 19 to 20, characterized in preparing an agent, which contains insulin and/or proinsulin and/or des-Phe-insulin and/or C peptide and insulin derivative of the formula I in combination with several different auxiliaries having delaying action.

**Revendications pour Les Etats contractants BE CH DE FR GB IT LI LU NL SE**

1. Médicament à base d'un véhicule physiologiquement acceptable et d'une combinaison de substances actives, caractérisé en ce qu'il contient en tant que combinaison de substances actives

a) un dérivé d'insuline de formule I

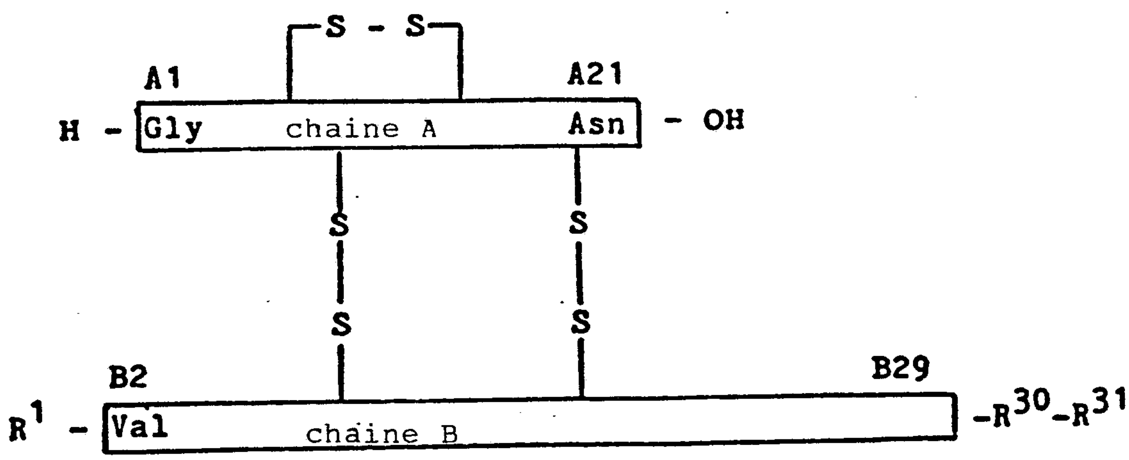

dans laquelle
$R^1$ représente H ou H-Phe,
$R^{30}$ représente le reste d'un acide L-aminé neutre, apte à être codé génétiquement, et
$R^{31}$ représente -OHG ou un groupe organique physiologiquement acceptable, à caractère basique, ayant jusqu'à 50 atomes de carbone, à l'édification duquel participent 0 à 3 acides α-aminés, et dont la fonction carboxy terminale éventuellement présente peut exister sous forme libre, sous forme de fonction ester, sous forme de fonction amide, sous forme de lactone, ou être réduite en $CH_2OH$, ayant un point isoélectrique situé entre 5,8 et 8,5, et

$b_1$) une insuline de formule I dans laquelle $R^1$ représente H ou H-Phe, $R^{30}$ représente Ala, Thr ou Ser, et $R^3$ représente OH, ou

$b_2$) des sels physiologiquement acceptables de

celle-ci, ou
$b_3$) la proinsuline, ou
$b_4$) le peptide C ou
$b_5$) une combinaison de $b_3$) et $b_4$),
le composant a) étant ajouté en une quantité d'au moins 1%.

2. Médicament selon la revendication 1, caractérisé en ce que dans le dérivé d'insuline de formule I mentionné en a),
$R^{31}$ représente un radical de formule $-X_n-S$, dans lequel
$n = 0, 1, 2$ ou 3,
X représente des restes identiques ou différents d'acides L-aminés neutres ou basiques existant dans la nature et/ou des acides D-aminés correspondant à ceux-ci, et S représente OH ou un groupement physiologiquement acceptable, bloquant le groupe carboxy, qui porte, lorsque $n = 0$, un radical basique apte à la protonation ou chargé positivement, ou qui peut porter, lorsque $n > 0$, un tel radical, et dans lequel la terminaison C -X-S peut également représenter le reste d'un acide aminé réduit en l'alcool correspondant, ou lorsque n = 2 ou 3, le reste homosérine-lactone.

3. Médicament selon la revendication 1 ou 2, caractérisé en ce que dans le dérivé d'insuline et dans l'insuline de formule I, $R^1$ représente H-Phe.

4. Médicament selon l'une des revendications 1 à 3, caractérisé en ce que dans le dérivé d'insuline de formule I mentionné en 1a), $R^{30}$ représente Ala, Thr ou Ser.

5. Médicament selon l'une des revendications 2 à 4, caractérisé en ce que dans le dérivé d'insuline de formule I mentionné en 1a), les restes d'acides aminés X sont présents sous la configuration L.

6. Médicament selon l'une des revendications 2 à 5, caractérisé en ce que dans le dérivé d'insuline de formule I mentionné en 1a), S représente OH, un groupe alcoxy en $C_1$–$C_6$, cycloalkyloxy en $C_3$–$C_6$, $NH_2$, dialkyl ($C_1$ - $C_6$)-amino, alkyl-($C_1$ - $C_6$)-amino, amino-alcoxy en $C_2$–$C_6$, alkyl($C_2$ - $C_4$)-amino-alcoxy($C_2$ - $C_6$), dialkyl($C_1$ - $C_4$)-amino-alcoxy ($C_2$–$C_6$), dialkyl($C_1$–$C_4$)-amino-alcoxy($C_2$–$C_6$), trialkyl($C_1$ - $C_4$)-ammonio-alcoxy($C_2$ - $C_6$), amino-alkyl($C_2$ - $C_6$)-amino, [alkyl($C_1$ - $C_4$)-amino]-alkyl($C_2$ - $C_6$)-amino, [dialkyl($C_1$ - $C_4$)-amino]-alkyl($C_2$ - $C_6$)-amino ou [trialkyl($C_1$ - $C_4$)-ammonio]-alkyl-($C_2$ - $C_6$)-amino.

7. Médicament selon l'une des revendications 2 à 6, caractérisé en ce que dans le dérivé d'insuline de formule I mentionnée en 1a), X représente un reste d'un acide aminé basique existant dans la nature, tel que Lys, Arg, His, Cit, Orn ou Hyl et/ou leurs formes D.

8. Médicament selon l'une des revendications 1 à 7, caractérisé en ce qu'il contient de l'insuline-B31-Arg-OH ou de l'insuline-B31-Arg-Arg-OH.

9. Médicament selon l'une des revendications 1 à 8, caractérisé en ce qu'il contient plusieurs dérivés d'insuline de formule I distincts et/ou plusieurs insulines de formule I distinctes.

10. Médicament selon l'une des revendications 1 à 9, caractérisé en ce qu'il contient une proinsuline ou un analogue de la proinsuline et/ou du peptide C humain.

11. Médicament selon l'une des revendications 1 à 10, caractérisé en ce qu'il contient du peptide C humain.

12. Médicament selon l'une des revendications 1 à 11, caractérisé en ce qu'il présente un pH entre 2,5 et 8,5, et se trouve à ce pH sous forme de solution ou de suspension, et contient un agent d'isotonie approprié en la quantité usuelle, ainsi qu'un agent de conservation convenable en quantité appropriée.

13. Médicament selon l'une des revendications 1 à 12, caractérisé en ce qu'il contient en outre une quantité appropriée d'une substance tampon appropriée, lorsque le PH est situé entre 5,0 et 8,5.

14. Médicament selon l'une des revendications 1 à 13, caractérisé en ce que la formulation contient une quantité appropriée d'un stabilisant approprié qui empêche la précipitation de la protéine lors d'une contrainte thermo-mécanique au contact avec des matériaux divers.

15. Médicament selon l'une des revendications 1 à 14, caractérisé en ce qu'il contient une quantité appropriée de zinc qui peut se situer entre 0 et 100 µg/100 unités.

16. Médicament selon l'une des revendications 1 à 15, caractérisé en ce que l'on utilise l'insulin et/ou la proinsuline et/ou la desPhe$^{B1}$-insuline et/ou le peptide C, et le dérivé d'insuline de formule I, sous forme d'un sel alcalin ou du sel d'ammonium.

17. Médicament selon l'une des revendications 1 à 16, caractérisé en ce que le composant insuline et/ou le composant proinsuline et/ou le composant desPhe$^{B1}$-insuline et/ou le composant peptide C, et le composant constitué par le dérivé d'insuline de formule I, peuvent se trouver chacun, indépendamment les uns des autres, sous forme dissoute, amorphe et/ou cristalline.

18. Médicament selon l'une des revendications 1 à 17, caractérisé en ce qu'une fraction quelconque, respectivement, du composant insuline et/ou du composant proinsuline et/ou du composant desPhe-insuline et/ou du composant peptide C, et du composant constitué par le dérivé d'insuline de formule I, se trouve sous forme cristalline, une autre fraction quelconque, respectivement, du composant insuline et/ou du composant proinsuline et/ou du composant desPhe-insuline et/ou du composant peptide C, et du composant constitué par le dérivé d'insuline de formule I, se trouve sous forme amorphe, et le reste, respectivement, du composant insuline et/ou de la proinsuline et/ou du composant des Phe-insuline et/ou du composant peptide C, et du composant constitué par le dérivé d'insuline de formule I, se trouve sous forme dissoute.

19. Médicament selon l'une des revendications 1 à 18, caractérisé en ce que la préparation contient en quantités appropriés un des adjuvants à action retardatrice connus.

20. Médicament selon la revendication 19, caractérisé en ce que l'on utilise ce principe retard

en combinaison avec l'ensemble des composants actifs ou avec des fractions de ceux-ci.

21. Médicament selon l'une des revendications 19 ou 20, caractérisé en ce qu'il contient de l'insuline et/ou de la proinsuline et/ou de la des-Phe-insuline et/ou du peptide C et un dérivé d'insuline de formule I, en combinaison avec plusieurs adjuvants à action retardatrice distincts.

22. Procédé pour la préparation d'un médicament selon l'une des revendications 1 à 21, caractérisé en ce que l'on met la combinaison de substances actives sous une forme d'administration appropriée.

23. Médicament selon l'une des revendications 1 à 21, à utiliser dans le traitement du diabète sucré.

**Revendications pour L'Etat contractant AT**

1. Procédé pour la préparation d'un médicament à partir d'un véhicule physiologiquement acceptable et d'une combinaison de substances actives à base de

a) un dérivé d'insuline de formule I

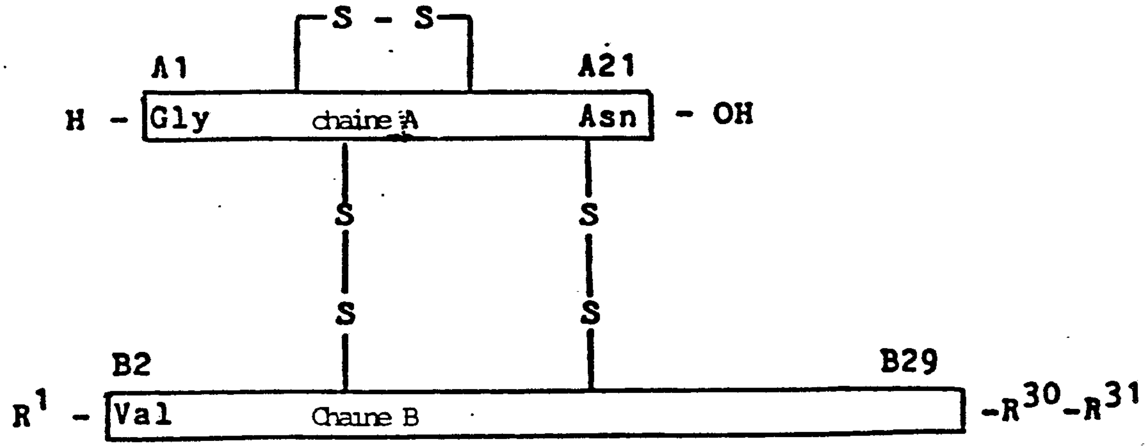

dans laquelle
$R^1$ représente H ou H-Phe,
$R^{30}$représente le reste d'un acide L-aminé neutre, apte à être codé génétiquement, et
$R^{31}$ représente -OHG ou un groupe organique physiologiquement acceptable, à caractère basique, ayant jusqu'à 50 atomes de carbone, à l'édification duquel participent 0 à 3 acides α-aminés, et dont la fonction carboxy terminale écentuellement présente peut exister sous forme libre, sous forme de fonction ester, sous forme de fonction amide, sous forme de lactone, ou être réduite en $CH_2OH$, ayant un point isoélectrique situé entre 5,8 et 8,5, et
$b_1$) une insuline de formule I dans laquelle $R^1$ représente H ou H-Phe, $R^{30}$ représente Ala, Thr ou Ser, et $R^3$ représente OH, ou
$b_2$) des sels physiologiquement acceptables de celle-ci, ou
$b_3$) la proinsuline, ou
$b_4$) le peptide C ou
$b_5$) une combinaison de $b_3$) et $b_4$),
le composant a) étant ajouté en une quantité d'au moins 1%, caractérisé en ce qu'on le met sous une forme d'administration appropriée.

2. Procédé selon la revendication 1, caractérisé en ce que l'on prépare un médicament dans lequel, dans le dérivé d'insuline de formule I mentionné en a),
$R^{31}$ représente un radical de formule $-X_n-S$,
dans lequel
n = 0, 1, 2 ou 3
X représente des restes identiques ou différents d'acides L-aminés neutres ou basiques existant dans la nature et/ou des acides D-aminés correspondant à ceux-ci, et S représente OH ou un groupement physiologiquement acceptable, bloquant le groupe carboxy, qui porte, lorsque n = 0, un radical basique apte à la protonation ou chargé positivement, ou qui peut porter, lorsque n > 0, un tel radical, et dans lequel la terminaison C -X-S peut également représenter le reste d'un acide aminé réduit en l'alcool correspondant, ou lorsque n = 2 ou 3, le reste homosérine-lactone.

3. Procédé selon la revendication 1 ou 2, caractérisé en ce que l'on prépare un médicament dans lequel, dans le dérivé d'insuline et dans l'insuline de formule I, $R^1$ représente H-Phe.

4. Procédé selon l'une des revendications 1 à 3, caractérisé en ce que l'on prépare un médicament dans lequel, dans le dérivé d'insuline de formule I mentionné en 1a), $R^{30}$ représente Ala, Thr ou Ser.

5. Procédé selon l'une des revendications 2 à 4, caractérisé en ce que l'on prépare un médicament dans lequel, dans le dérivé d'insuline de formule I mentionné en 1a), les restes d'acides aminés X se trouvent sous la configuration L.

6. Procédé selon l'une des revendications 2 à 5, caractérisé en ce que l'on prépare un médicament dans lequel, dans le dérivé d'insuline de formule I mentionné en 1a), S représente OH, un groupe alcoxy en $C_1$-$C_6$, cycloalkyloxy en $C_3$-$C_6$, $NH_2$, dialkyl ($C_1$ - $C_6$)-amino, alkyl-($C_1$ - $C_6$)-amino, amino-alcoxy en $C_2$-$C_6$, alkyl($C_2$ - $C_4$)-amino-alcoxy($C_2$ - $C_6$), dialkyl($C_1$ - $C_4$)-amino-alcoxy ($C_2$-$C_6$), trialkyl($C_1$ - $C_4$)-ammonio-alcoxy($C_2$ - $C_6$), amino-alkyl($C_2$ - $C_6$)-amino, [alkyl($C_1$ - $C_4$)-amino]-alkyl($C_2$ - $C_6$)-amino, [dia-

lkyl($C_1$ - $C_4$)-amino]-alkyl($C_2$ - $C_6$)-amino ou [trialkyl($C_1$ - $C_4$)-ammonio]-alkyl-($C_2$ - $C_6$)-amino.

7. Procédé selon l'une des revendications 2 à 6, caractérisé en ce que l'on prépare un médicament dans lequel, dans le dérivé d'insuline de formule I mentionnée en 1a), X représente un reste d'un acide aminé basique existant dans la nature, tel que Lys, Arg, His, Cit, Orn ou Hyl et/ou leurs formes D.

8. Procédé selon l'une des revendications 1 à 7, caractérisé en ce que l'on prépare un médicament qui contient de l'insuline-B31-Arg-OH ou de l'insuline-B31-Arg-Arg-OH.

9. Procédé selon l'une des revendications 1 à 8, caractérisé en ce l'on prépare un médicament qui contient plusieurs dérivés d'insuline de formule I distincts et/ou plusieurs insulines de formule I distinctes.

10. Procédé selon l'une des revendications 1 à 9, caractérisé en ce l'on prépare un médicament qui contient une proinsuline ou un analogue de la proinsuline et/ou du peptide C humain.

11. Procédé selon l'une l'une des revendications 1 à 10, caractérisé en ce l'on prépare un médicament qui contient du peptide C humain.

12. Procédé selon l'une des revendications 1 à 11, caractérisé en ce l'on prépare un médicament qui présente un pH entre 2,5 et 8,5, et se trouve à ce pH sous forme de solution ou de suspension, et contient un agent d'isotonie approptié en la quantité usuelle, ainsi qu'un agent de conservation convenable en quantité appropriée.

13. Procédé selon l'une des revendications 1 à 12, caractérisé en ce l'on prépare un médicament qui contient en outre une quantité appropriée d'une substance tampon appropriée, lorsque le PH est situé entre 5,0 et 8,5.

14. Procédé selon l'une des revendications 1 à 13, caractérisé en ce que l'on prépare un médicament qui contient une quantité appropriée d'un stabilisant approprié qui empêche la précipitation de la protéine lors d'une contrainte thermomécanique au contact avec des matériaux divers.

15. Procédé selon l'une des revendications 1 à 14, caractérisé en ce l'on prépare un médicament qui contient une quantité appropriée de zinc qui peut se situer entre 0 et 100 µg/100 unités.

16. Procédé selon l'une des revendications 1 à 15, caractérisé en ce que l'on prépare un médicament dans lequel on utilise l'insuline et/ou la proinsuline et/ou la desPhe$^{B1}$-insuline et/ou le peptide C, et le dérícé d'insuline de formule I, sous forme d'un sel alcalin ou du sel d'ammonium.

17. Procédé selon l'une des revendications 1 à 16, caractérisé en ce que l'on prépare un médicament dans lequel le composant insuline et/ou le composant proinsuline et/ou le composant desPhe$^{B1}$-insuline et/ou le composant peptide C, et le composant constitué par le dérivé d'insuline de formule I, peuvent se trouver chacun, indépendamment les uns des autres, sous forme dissoute, amorphe et/ou cristalline.

18. Procédé selon l'une des revendications 1 à 17, caractérisé en ce l'on prépare un médicament dans lequel une fraction quelconque, respectivement, du composant insuline et/ou du composant proinsuline et/ou du composant desPhe-insuline et/ou du composant peptide C, et du composant constitué par le dérícé d'insuline de formule I, se trouve sous forme cristalline, une autre fraction quelconque, respectivement, du composant insuline et/ou du composant proinsuline et/ou du composant desPhe-insuline et/ou du composant peptide C, et du composant constitué par le dérivé d'insuline de formule I, se trouve sous forme amorphe, et le reste, respectivement, du composant insuline et/ou de la proinsuline et/ou du composant desPhe-insuline et/ou du composant peptide C, et du composant constitué par le dérivé d'insuline de formule I, se trouve sous forme dissoute.

19. Procédé selon l'une des revendications 1 à 18, caractérisé en ce que l'on prépare un médicament qui contient en quantités appropriés un des adjuvants à action retardatrice connus.

20. Procédé selon la revendication 19, caractérisé en ce que l'on utilise ce principe retard en combinaison avec l'ensemble des composants actifs ou avec des fractions de ceux-ci.

21. Procédé selon l'une des revendications 19 ou 20, caractérisé en ce que l'on prépare un médicament qui contient de l'insuline et/ou de la proinsuline et/ou de la des Pheinsuline et/ou du peptide C et un dérivé d'insuline de formule I, en combinaison avec plusieurs adjuvants à action retardatrice distincts.